(19) Europäisches Patentamt
European Patent Office
Office européen des brevets

(11) **EP 4 031 079 B1**

(12) **EUROPEAN PATENT SPECIFICATION**

(45) Date of publication and mention
of the grant of the patent:
**09.10.2024  Bulletin 2024/41**

(21) Application number: **20864676.0**

(22) Date of filing: **15.09.2020**

(51) International Patent Classification (IPC):
***A61F 5/01*** *(2006.01)*         ***A61F 7/00*** *(2006.01)*
***A61F 7/02*** *(2006.01)*

(52) Cooperative Patent Classification (CPC):
**A61F 5/0106; A61F 5/0123; A61F 7/007;**
A61F 2007/0042; A61F 2007/0075; H10N 10/00

(86) International application number:
**PCT/IL2020/051006**

(87) International publication number:
**WO 2021/053663 (25.03.2021 Gazette 2021/12)**

(54) **ORTHOPEDIC BRACE**

ORTHOPÄDISCHE STÜTZE

ATTELLE ORTHOPÉDIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **16.09.2019   US 201962900660 P**

(43) Date of publication of application:
**27.07.2022   Bulletin 2022/30**

(73) Proprietor: **Coral Star Medical Ltd.
2010800 Kfar Hanania (IL)**

(72) Inventors:
• **GENGRINOVITCH, Almog
2164128 Karmiel (IL)**

• **GENGRINOVITCH, Stela
2010800 Kfar Hanania (IL)**

(74) Representative: **Straus, Alexander
2K Patent- und Rechtsanwälte
Bajuwarenring 14
82041 Oberhaching (DE)**

(56) References cited:
**WO-A1-2015/079237        WO-A1-2018/102182
WO-A1-2018/138537        JP-A- 2017 018 409
US-A1- 2005 075 593       US-A1- 2011 071 603
US-A1- 2019 015 289       US-A1- 2019 099 287**

EP 4 031 079 B1

# Description

## FIELD OF THE INVENTION

The present invention relates to an orthopedic brace including therein a thermal treatment system useful for treating joint injuries and musculoskeletal disorders, such as osteoarthritis.

## BACKGROUND OF THE INVENTION

Osteoarthritis is a chronic progressive disease that causes disability, particularly among the elderly. The disease is characterized by the breakdown of cartilage in the joints which in turn leads to various complications, including, pain and impaired movement.

Orthopedic braces involve one of the approaches for alleviating and/or treating complications associated with osteoarthritis.

For example, WO02/000159 discloses magnetic devices and use thereof for the relief of pain, by positioning the devices in on the organ/tissue of interest.

WO 03/090868 discloses an apparatus for positioning one or more ultrasonic transducers with respect to a joint for delivery of ultrasonic therapy thereto.

US 2018/0056069 discloses an electroacupuncture device for treating osteoarthritis in a patient.

US 10,226,640 discloses a lightweight, wearable, battery-operated electromagnetic field therapy system for treating degenerative joint disease.

Heating or cooling the joint have been used for the treatment of osteoarthritis and other orthopedic complications, as disclosed for example in US 5,314,455; US 5,466,250; US 7,243,509; US 8,043,242; US 9,808,395; WO 2005/007060 and US 2014/0046232.

WO 2015/079237 describes an orthopaedic device which comprises a thermoelectric module capable of generating heat and providing a cooling effect to a region of a subject fitted with the device. The device includes a detachable fan, and is configured to cause the thermoelectric module to generate heat when the fan is not attached to the thermoelectric module, and the device is configured to cause the thermoelectric module to provide a cooling effect when the fan is attached to the thermoelectric module.

US 2019/0015289 describes a treatment apparatus for applying combinations of thermal and compressive treatment programmes to a human or animal body which comprises a brace which itself comprises first and second brace portions connected by webbing/strapping. The strapping comprises first and second straps, each of which engages both of the brace portions via a loop on one of the brace portions and is held by an anchor on the other brace portion. The brace portions each comprise a rigid outer shell of moulded plastics material, and each includes a mounting socket for detachably mounting a thermal treatment unit. The mounting sockets include apertures that extend through the brace portions to the patient facing side, so as to present an active face of the treatment unit to the treatment site.

WO 2018/102182 describes a wearable cooling and heating system for placement at a body region of a user, including a retention mechanism defining an internal side, and an external side; a temperature modulation subsystem, coupled to the retention mechanism, including a temperature- controllable substrate arranged at the internal side of the retention mechanism, a shield, defining a shield cavity, the shield arranged at the external side of the retention mechanism and coupled to the temperature-controllable substrate, and a heat exchanger in thermal contact with the temperature-controllable substrate and arranged within the shield cavity; a control module, retained by the retention mechanism, communicatively coupled to the temperature modulation subsystem, wherein the temperature modulation subsystem is operable between a cooling mode and a heating mode by the control module, wherein the cooling mode includes generating a low temperature at the temperature-controllable substrate, and wherein the heating mode includes generating a high temperature at the temperature-controllable substrate.

WO 2018/0138537 describes an active hand orthosis for people with nerve-related paresis/paralysis of the hand, and for people with spastic paresis/paralysis of the hand, which comprises supporting elements and moving elements connecting the supporting elements, the moving elements containing shape memory material, further comprising a control system, electric circuit elements and a power source for creating an activation signal inducing transformation of the shape memory material, and thereby deformation of the moving elements, wherein the moving elements comprise: a wrist joint moving element, at least one finger moving element, and a thumb moving element, furthermore, in a state fitted to a lower arm the supporting elements comprise: a lower arm splint supporting a lower side of the lower arm, and leaving a wrist joint free, a palm support connected to the lower arm splint by the wrist j oint moving element, palm support being provided at the proximal end of the fingers opposable to the thumb, at least one finger support connected to the palm support by the at least one finger moving element, at least one finger support being provided at the middle bone of the fingers opposable to the thumb, and a thumb support connected to the lower arm splint by the thumb moving element, the thumb support extending from the proximal end of the thumb on the palm to the lower bone of the thumb.

There still remains an unmet need for improved methods and devices for treating joint diseases and disorders, particularly osteoarthritis.

## SUMMARY OF THE INVENTION

The invention is defined by independent claim 1.
Advantageous embodiments are defined by the dependent claims.

The present invention generally relates to the field of orthopedic braces to be worn in the vicinity of a joint. Specifically, the present invention provides orthopedic braces, which are especially beneficial in treating joint related diseases and/or elevating pain associated with joint damages. Specifically, the orthopedic brace of the present invention may be used for treating joint injuries and musculoskeletal disorders including osteoarthritis and degenerative diseases of the joints, as well as joint pain, tenderness, limitation of movement, crepitus (grating, cracking or popping sounds in the joint), stiffness after immobility, and joint inflammation.

According to some embodiments, there is provided an orthopedic brace configured to provide a heat-based treatment to a joint of a subject in need thereof. The orthopedic brace of the present invention is worn on the subject's body in the vicinity of the joint and includes at least one housing, at least one thermoelectric cooler (TEC) and at least one heat transferring medium. A main component of the present orthopedic brace is the thermoelectric cooler. In general, and as detailed below with respect to TECs, the thermoelectric cooler of the present orthopedic brace is an electronic device, which is flat and has two faces, wherein upon application of electric current thereto, one of its faces is heated up and the other face is cooled down with respect to the ambient temperature. As further detailed below, the determination of the hot and cold face of the TEC depends upon the construction and assembly of the TEC and further upon the polarity of electricity operating it, according to some embodiments, which may allow temperature control. While the thermoelectric cooler is the source of temperature control of the present orthopedic brace, it is generally not in direct contact with the subject's body, according to some embodiments. Specifically, one face of the thermoelectric cooler is thermally coupled to a heat transferring medium comprising a thermally conductive material. The medium is in direct or indirect (e.g. through appropriate padding) with the subject's body when worn, and thus is configured to conduct heat between the subject's body and the thermoelectric cooler to achieve the desired thermal treatment to the joint. The housing of the present orthopedic brace generally serves for structural adaptation to the subject's body and for housing of components of the orthopedic brace.

One of the advantages of using a thermoelectric cooler is the rapid temperature changes it can provide, according to some embodiments. Because of these rapid temperature modifications the thermoelectric cooler is preferably housed within a housing, which may thermally insulate the thermoelectric cooler from the subject, according to some embodiments. In addition, it is preferred that the thermoelectric cooler is not in direct contact with the subject's body, but rather through a mediator in the heat transferring medium.

Another beneficial optional feature of the present orthopedic brace is that its heat transferring medium may be a semi-solid, wherein upon temperature modification it may at least temporarily harden (e.g. solidify when cold) to fix the joint and the bones connected thereby in a predetermined conformation. This feature may enhance the treatment when fixation of the joint is beneficial.

Thus, according to some embodiments, there is provided an orthopedic brace comprising at least one housing having an internal face and an external face, wherein said internal face is structured to be worn by a subject, in a vicinity of a joint; at least one substantially flat thermoelectric cooler (TEC) disposed at least partially within the housing, and having an internal TEC face and an external TEC face, wherein the external TEC face is facing the external face of said housing; and at least one heat transferring medium coupled to the thermoelectric cooler and comprising a first thermally conductive material, wherein the heat transferring medium has an external face, facing and thermally coupled to the internal TEC face, and an internal face; wherein upon application of electrical current to the thermoelectric cooler, the temperature of the external TEC face is being either elevated or lowered and the temperature of the internal TEC face is being either lowered or elevated in the opposite direction, thereby lowering or elevating the temperature of the heat transferring medium.

According to some embodiments, the internal heat transferring medium face is facing the joint, when the orthopedic brace is worn by the subject.

According to some embodiments, the internal TEC face is facing the joint, when the orthopedic brace is worn by the subject.

According to some embodiments, the first thermally conductive material is selected from the group consisting of a thermally conductive gel, thermally conductive solid, a thermally conductive liquid, a thermally conductive solution, a thermally conductive emulsion and a thermally conductive suspension. Each option represents a separate embodiment.

According to some embodiments, the thermally conductive material is a thermally conductive semi solid.

According to some embodiments, lowering the temperature of the heat transferring medium comprises setting the temperature of heat transferring medium at a first temperature. According to some embodiments, the first temperature is lower than ambient temperature. According to some embodiments, at the first temperature the thermally conductive material is harder than it is at ambient temperature. According to some embodiments, the heat transferring medium is substantially rigid at the first temperature. According to some embodiments, the heat transferring medium is substantially solid at the first temperature. According to some embodiments, the heat transferring medium is in a non-bendable state when in the first temperature.

According to some embodiments, the heat transferring medium is in a bendable state when in room temperature. According to some embodiments, the heat transferring medium is substantially flexible at room temperature. According to some embodiments, the heat transferring medium is substantially pliable at the first temperature. According to some embodiments, upon application of electrical current to the thermoelectric cooler the temperature of the heat transferring medium is either lowered or elevated, thereby the heat transferring medium being in either the non-bendable state or the bendable state respectively. According to some embodiments, the thermoelectric cooler is configured to cool the heat transferring medium thereby to transform it from the bendable state to the non-bendable state.

According to some embodiments, when the orthopedic brace is worn by the subject and when in the heat transferring medium is in a non-bendable state, the joint is substantially fixed in a predetermined conformation. According to some embodiments, when the orthopedic brace is worn by the subject and when in the heat transferring medium is in a bendable state, the joint is substantially movable.

According to some embodiments, the thermally conductive material is a thermally conductive gel. According to some embodiments, the heat transferring medium is a gel bag.

According to some embodiments, the gel bag has a depth, measured between the gel bag external face and the gel bag internal face. According to some embodiments, each of the gel bag external face and the gel bag internal face has a substantially equal area having a length and a width. According to some embodiments, each of the length and a width is at least 2.5 times greater than the gel bag depth. According to some embodiments, the gel bag depth is in the range of 2 mm to 20 mm. According to some embodiments, each of the gel bag external face area and the gel bag internal face area has length and width, each in the range of 10 mm to 250 mm.

According to some embodiments, the heat transferring medium is coupled to the housing. According to some embodiments, the heat transferring medium is connected to the housing.

According to some embodiments, the heat transferring medium is at least partially disposed within the housing.

According to some embodiments, the first thermally conductive material has a thermal conductivity in the range of 0.5 to 24,000 $(W*m^{-1}*K^{-1})$

According to some embodiments, the orthopedic brace further comprises a substantially flat heat transferring connector. According to some embodiments, the heat transferring connector is made of a second thermally conductive material. According to some embodiments, the heat transferring connector has an external face coupled to the thermoelectric cooler. According to some embodiments, the heat transferring connector has an internal face contacting the heat transferring medium. According to some embodiments, the orthopedic brace further comprises a substantially flat heat transferring connector made of a second thermally conductive material, and having an external face coupled to the thermoelectric cooler and an internal face contacting the heat transferring medium.

According to some embodiments, the second thermally conductive material comprises a metal, a metal alloy or a combination thereof.

According to some embodiments, the external face of the heat transferring connector is connected to the internal TEC face through a first thermal paste disposed there between. According to some embodiments, the first thermal paste has a thermal conductivity in the range of 0.5 to 100 $W*m^{-1}*K^{-1}$.

According to some embodiments, upon application of electrical current to the thermoelectric cooler, the temperature of the internal TEC face is being either lowered or elevated, thereby lowering or elevating the temperature of the heat transferring connector respectively. According to some embodiments, upon the temperature of the heat transferring connector being either lowered or elevated, heat is conducted between the heat transferring connector and the heat transferring medium to lower or elevate the temperature of the heat transferring medium respectively. According to some embodiments, upon application of electrical current to the thermoelectric cooler, the temperature of the internal TEC face is being either lowered or elevated, thereby lowering or elevating the temperature of the heat transferring connector respectively, and wherein upon the temperature of the heat transferring connector being either lowered or elevated, heat is conducted between the heat transferring connector and the heat transferring medium to lower or elevate the temperature of the heat transferring medium respectively.

According to some embodiments, the orthopedic brace further comprises a control unit, electrically coupled to the thermoelectric cooler. According to some embodiments, the control unit is configured to monitor the electrical current applied thereto. According to some embodiments, the control unit is configured to monitor the electrical current applied thereto from a power source. According to some embodiments, the orthopedic brace further comprises a control unit, electrically coupled to the thermoelectric cooler and configured to monitor the electrical current applied thereto.

According to some embodiments, the control unit is disposed within the housing.

According to some embodiments, the control unit comprises an H-bridge configured to switch the direction of electrical current applied to the thermoelectric cooler between a first direction and an opposite direction. According to some embodiments, the H-bridge is configured to switch the voltage polarity applied to the thermoelectric cooler. According to some embodiments, upon application of electrical current in the first direction to the thermoelectric cooler, the temperature of the external TEC face is being elevated and the temperature of the internal TEC face is being lowered, thereby lowering the temperature of the heat transferring medium. According to some embodiments, upon application of electrical current in the opposite direction to the thermoelectric cooler, the temperature of the external TEC face is being lowered and the temperature of the internal TEC face is being elevated, thereby elevating the temperature of the heat transferring medium. According to some embodiments, the control unit comprises an H-bridge configured to switch the direction of electrical current applied to the thermoelectric cooler between a first direction and an opposite direction, wherein upon application of electrical current in the first direction to the thermoelectric cooler, the temperature of the external TEC face is being elevated and the temperature of the internal TEC face is being lowered, thereby lowering the temperature of the heat transferring medium, and wherein upon application of electrical current in the opposite direction to the thermoelectric cooler, the temperature of the external TEC face is being lowered and the temperature of the internal TEC face is being elevated, thereby elevating the temperature of the heat transferring medium.

According to some embodiments, the orthopedic brace further comprises a cooling unit configured to evacuate heat from the external TEC face.

According to some embodiments, the cooling unit comprises a heat sink coupled to the thermoelectric cooler.

According to some embodiments, the external TEC face is connected to the heat sink, through a second thermal paste disposed there between. According to some embodiments, the second thermal paste has a thermal conductivity in the range of 0.5 to 100 $W*m^{-1}*K^{-1}$.

According to some embodiments, the heat sink comprises a platform having an internal face and an external face. According to some embodiments, the internal face comprises a recess dimensioned to accommodate the external TEC face. According to some embodiments, the internal heat sink face is connected to the recess. According to some embodiments, the internal heat sink face is connected to the recess through a second thermal paste disposed therein. According to some embodiments, the heat sink further comprises a plurality of fins, each extending externally from the external heat sink face. According to some embodiments, the heat sink comprises a platform having an internal face and an external face, wherein the internal face comprises a recess dimensioned to accommodate the external TEC face, wherein the internal heat sink face is connected to the recess, through a second thermal paste disposed therein, wherein the heat sink further comprises a plurality of fins, each extending externally from the external heat sink face.

According to some embodiments, the heat sink is made of a heat conductive material. According to some embodiments, the heat sink is made of a heat conductive material having a thermal conductivity in the range of 7 to 24,000 $W*m^{-1}*K^{-1}$.

According to some embodiments, wherein the cooling unit further comprises a fan configured and position to create air flow in the direction of the heat sink, thereby evacuating heat therefrom.

According to some embodiments, the orthopedic brace further comprising at least one temperature sensor. According to some embodiments, the temperature sensor is positioned in the vicinity of the heat transferring medium. According to some embodiments, the temperature sensor is configured to measure the temperature of the heat transferring medium. According to some embodiments, the temperature sensor is configured to send to the control unit temperature indicating signals indicative of the temperature of the heat transferring medium. According to some embodiments, the orthopedic brace further comprising at least one temperature sensor positioned in the vicinity of the heat transferring medium and configured to measure the temperature thereof and further configured to send to the control unit temperature indicating signals indicative of the temperature of the heat transferring medium.

According to some embodiments, the control unit is configured to control the temperature of the heat transferring medium based on the temperature thereof. According to some embodiments, the control unit is configured to control the temperature of the heat transferring medium based on the temperature thereof, through monitoring the electrical current applied thereto.

According to some embodiments, the control unit comprises at least one predetermined operation program. According to some embodiments, the operation program comprises instruction that when executed by the control unit bring the temperature of the heat transferring medium to a first predetermined temperature for a first predetermined period of time. According to some embodiments, the operation program comprises instruction that when executed by the control unit bring the temperature of the heat transferring medium to a first predetermined temperature for a first predetermined period of time through controlling the current applied to the thermoelectric cooler. According to some embodiments, the operation program comprises instruction that when executed by the control unit bring the temperature of the heat transferring medium to a first predetermined temperature for a first predetermined period of time through controlling the current applied to the thermoelectric cooler and based on the temperature indicating signals. According to some embodiments, the control unit comprises at least one predetermined operation program, the program comprising bringing the temperature of the heat transferring medium to a first predetermined temperature for a first predetermined period of time through controlling the current applied to the thermoelectric cooler and based on the temperature indicating signals.

According to some embodiments, the program further comprises instructions that when executed by the control unit bring the temperature of the heat transferring medium to a second predetermined temperature for a second predetermined period of time. According to some embodiments, the program further comprises instructions that when executed by the control unit bring the temperature of the heat transferring medium to a second predetermined temperature for a second predetermined period of time through controlling the current applied to the thermoelectric cooler. According to some embodiments, the program further comprises instructions that when executed by the control unit bring the temperature of the heat transferring medium to a second predetermined temperature for a second predetermined period of time through controlling the current applied to the thermoelectric cooler and based on the temperature indicating signals. According to some embodiments, the program further comprises bringing the temperature of the heat transferring medium to a second predetermined temperature for a second predetermined period of time through controlling the current applied to the thermoelectric cooler and based on the temperature indicating signals.

According to some embodiments, the control unit comprises a plurality of predetermined operation programs. According to some embodiments, each program comprises instructions that when executed by the control unit bring the temperature of the heat transferring medium to a predetermined temperature for a predetermined period of time. According to some embodiments, each program comprises instructions that when executed by the control unit bring the temperature of the heat transferring medium to a predetermined temperature for a predetermined period of time through controlling the current applied to the thermoelectric cooler. According to some embodiments, each program comprises instructions that when executed by the control unit bring the temperature of the heat transferring medium to a predetermined temperature for a predetermined period of time through controlling the current applied to the thermoelectric cooler and based on the temperature indicating signals. According to some embodiments, the control unit comprises a plurality of predetermined operation programs, each program comprising bringing the temperature of the heat transferring medium to a predetermined temperature for a predetermined period of time through controlling the current applied to the thermoelectric cooler and based on the temperature indicating signals.

According to some embodiments, the orthopedic brace further comprises a control panel. According to some embodiments, the control panel is operatively coupled to the control unit. According to some embodiments, the control panel is configured to send instruction signals to the control unit. According to some embodiments, the instruction signals comprise: instructions to control the temperature of the heat transferring medium, instructions to initiate at least one predetermined operation program, select one of the plurality of predetermined operation programs or a combination thereof. Each possibility represents a separate embodiment. According to some embodiments, the orthopedic brace further comprises a control panel operatively coupled to the control unit and configured to send instruction signals thereto, wherein the instruction signals comprise: instructions to control the temperature of the heat transferring medium, instructions to initiate at least one predetermined operation program, select one of the plurality of predetermined operation programs or a combination thereof.

According to some embodiments, the control panel is connected to an outer surface of the housing.

According to some embodiments, the control unit is configured to send indication signals to the control panel. According to some embodiments, the control panel is configured to display indications indicated by said indication signals. According to some embodiments, the indication signals comprise: indication of the operation of the control unit, indication of the temperature of the heat transferring medium, indication of a progression of a predetermined program or a combination thereof. Each possibility represents a separate embodiment. According to some embodiments, the control unit is configured to send indication signals to the control panel, wherein the control panel is configured to display indications indicated by said indication signals, wherein the indication signals comprise: indication of the operation of the control unit, indication of the temperature of the heat transferring medium, indication of a progression of a predetermined program and a combination thereof.

According to some embodiments, the control unit is configured to receive wireless instruction signals from an external processing unit. According to some embodiments, the instruction signals comprise: instructions to control the temperature of the heat transferring medium, instructions to initiate at least one predetermined operation select one of the plurality of predetermined operation programs or a combination thereof. Each possibility represents a separate embodiment. According to some embodiments, the control unit is configured to receive wireless instruction signals from an external processing unit, wherein the instruction signals comprise: instructions to control the temperature of the heat transferring medium, instructions to initiate at least one predetermined operation select one of the plurality of predetermined operation programs or a combination thereof.

According to some embodiments, the control unit is configured to send wireless indication signals to the external processing unit. According to some embodiments, the processing unit is configured to display indications indicated by said indication signals through a display unit associated therewith. According to some embodiments, the indication signals comprise: indication of the operation of the control unit, indication of the temperature of the heat transferring medium, indication of a progression of a predetermined program and a combination thereof. Each possibility represents a separate embodiment. According to some embodiments, the control unit is configured to send wireless indication signals to the external processing unit, wherein the processing unit is configured to display indications indicated by said indication signals through a display unit associated therewith, wherein the indication signals comprise: indication of the operation of the control unit, indication of the temperature of the heat transferring medium, indication of a progression of a predetermined program and a combination thereof.

According to some embodiments, the orthopedic brace further comprises a vibrating unit. According to some embodiments, the vibrating unit is positioned on the internal face of the housing. According to some embodiments, the orthopedic brace further comprises a vibrating unit positioned on the internal face of the housing. According to some embodiments, the vibrating unit is positioned in the vicinity of the joint when the orthopedic brace is worn by the subject. According to some embodiments, the vibrating unit is positioned in the vicinity of the heat transferring medium. According to some embodiments, the vibrating unit is contacting the heat transferring medium.

According to some embodiments, the vibrating unit comprises a vibration motor, electrically coupled to the control unit and configured to create a vibration upon application of electric current thereby.

According to some embodiments, the orthopedic brace comprises: a first housing having a first housing internal face and a first housing external face, wherein said first housing internal face is structured to be worn by the subject, in the vicinity of the joint; a second housing having a second housing internal face and a second housing external face, wherein said second housing internal face is structured to be worn by the subject, in the vicinity of the same joint; a first substantially flat thermoelectric cooler (TEC) disposed at least partially within the first housing, and having a first internal TEC face and a first external TEC face, wherein the first external TEC face is facing the external face of said housing;

a second substantially flat thermoelectric cooler (TEC) disposed at least partially within the second housing, and having a second internal TEC face and a second external TEC face, wherein the second external TEC face is facing the external face of said housing; at least one heat transferring medium coupled to at least one of the first thermoelectric cooler and the second thermoelectric cooler, and comprising a first thermally conductive material, wherein the at least one heat transferring medium has an external face, facing and thermally coupled to at least one of the first internal TEC face and the second internal TEC face, and a heat transferring medium internal face; wherein upon application of electrical current to each one of the first thermoelectric cooler and second thermoelectric cooler, the temperature of the corresponding external TEC face is being either elevated or lowered and the temperature of the corresponding internal TEC face is being either lowered or elevated in the opposite direction, thereby lowering or elevating the temperature of the heat transferring medium thermally coupled thereto.

According to some embodiments, the orthopedic brace comprises: a first heat transferring medium coupled to the first thermoelectric cooler and comprising a thermally conductive material, wherein the first heat transferring medium has a first heat transferring medium external face, facing and thermally coupled to the first internal TEC face, and a first heat transferring medium internal face; and a second heat transferring medium coupled to the second thermoelectric cooler and comprising a thermally conductive material, wherein the second heat transferring medium has a second heat transferring medium external face, facing and thermally coupled to the second internal TEC face, and a second heat transferring medium internal face; wherein upon application of electrical current to the first thermoelectric cooler, the temperature of the first external TEC face is being either elevated or lowered and the temperature of the first internal TEC face is being either lowered or elevated in the opposite direction, thereby lowering or elevating the temperature of the first heat transferring medium; and wherein upon application of electrical current to the second thermoelectric cooler, the temperature of the second external TEC face is being either elevated or lowered and the temperature of the second internal TEC face is being either lowered or elevated in the opposite direction, thereby lowering or elevating the temperature of the second heat transferring medium.

According to some embodiments, the orthopedic brace comprises a single heat transferring medium extending between the first housing and the second housing and having a first portion coupled to the first thermoelectric cooler and a second portion coupled to the second thermoelectric cooler, wherein the single heat transferring medium comprises a first thermally conductive material, wherein the first portion has a first portion external face, facing and thermally coupled to the first internal TEC face and a first portion internal face, wherein the second portion has a second portion external face, facing and thermally coupled to the second internal TEC face and a second portion internal face; wherein upon application of electrical current to each one of the first thermoelectric cooler and second thermoelectric cooler, the temperature of the corresponding external TEC face is being either elevated or lowered and the temperature of the corresponding internal TEC face is being either lowered or elevated in the opposite direction, thereby lowering or elevating the temperature of the first or second portion, coupled thereto.

According to some embodiments, the orthopedic brace comprises a control unit disposed within one of the first and second housings. According to some embodiments, the control unit is electrically coupled to each one of the first and second thermoelectric coolers and configured to monitor the electrical current applied thereto. According to some embodiments, the orthopedic brace comprises a control unit disposed within one of the first and second housings, wherein the control unit is electrically coupled to each one of the first and second thermoelectric coolers and configured to monitor the electrical current applied thereto.

According to some embodiments, the first housing comprises a first strap holder, extending externally from its external face, and the second housing comprises a second strap holder, extending externally from its external face.

According to some embodiments, the orthopedic brace further comprises a strap configured to connect between the first housing and the second housing through the first strap holder and the second strap holder, and further configured to adjust the orthopedic brace to be worn by the subject.

According to some embodiments, the thermoelectric cooler has power consumption in the range of 1 to 120 Watts.

According to some embodiments, each one of the internal TEC face and the external TEC face has a smooth surface.

According to some embodiments, there is provided a method for treating a disorder, disease or an injury of a joint of a subject, wherein the joint is selected from the group consisting of a knee joint, an elbow joint, a wrist joint, an ankle joint, a shoulder joint, a hip joint, a spinej oint, a fingerj oint, and a toe j oint, the method comprising applying the orthopedic brace of the present invention onto the joint.

Other objects, features and advantages of the present invention will become clear from the following description, examples and drawings.

**Brief Description of the Drawings**

Figures 1A-B show views in perspective of an orthopedic brace from an external viewpoint (Figure 1A) and from an internal viewpoint (Figure 1B), according to some embodiments.

Figures 2A-B show views in perspective of orthopedic braces when worn on a subject's leg, according to some embodiments.

Figures 3A-B show exploded assemblies of a thermoelectric cooler, a heat transferring connector and a heat transferring medium, according to some embodiments.

Figure 4 shows a view in perspective of an assembly of a thermoelectric cooler, a heat transferring connector and a heat transferring medium, according to some embodiments.

Figures 5A-B show cross sectional views of assemblies of a thermoelectric cooler, a heat transferring connector and a heat transferring medium, according to some embodiments.

Figures 6A-B show views in perspective of heat transferring media, according to some embodiments.

Figures 7A-B show views in perspective of two sets, each set including a first heat transferring medium and a matching second heat transferring medium, according to some embodiments.

Figure 8 shows a view in perspective of a U-shaped heat transferring medium, according to some embodiments.

Figure 9 shows a view in perspective of a thermoelectric cooler, according to some embodiments.

Figures 10A-B show views in perspective of two heat transferring connectors, according to some embodiments.

Figure 11 shows an exploded view of an orthopedic brace, according to some embodiments.

Figures 12A-B show views in perspective of a heat sink from an external view (Figure 12A) and from an internal view (Figure 12B), according to some embodiments.

Figure 13 shows a view in perspective of a fan and an air flow direction, according to some embodiments.

**Detailed Description**

Osteoarthritis is a chronic progressive disease that causes disability, particularly among the elderly. The disease is characterized by the breakdown of cartilage in the joints. Cartilage deterioration leads to bones rubbing against one another, causing pain, stiffness, and swelling, which impairs movement.

Osteoarthritis can also result in damaged ligaments, menisci, and muscles. It is estimated that about 10%-15% of adults over the age of sixty have some degree of osteoarthritis. Osteoarthritis mainly affects the joints of the knee, hands, feet, and spine, but is also common in other joints such as the shoulder and hip.

There are two types of osteoarthritis: primary and secondary. Primary osteoarthritis is a chronic degenerative disease that is related to aging. Secondary arthritis correlates with younger age, and is related to specific causes such as injury, occupation, diabetes, or obesity. Although the cause is different, the resulting pathology is the same. According to the World Health Organization (WHO), osteoarthritis is already one of the ten most disabling diseases in developed countries. About 10% of men and 18% of women over the age of 60 have symptomatic osteoarthritis worldwide, and about 80% of those have movement limitations, and 25% cannot perform their daily life activities.

Systemic risk factors associated with osteoarthritis include: age, gender, ethnicity, genetics, congenital/developmental conditions and diet. Age is one of the most common risk factors for osteoarthritis of all joints. Women are more likely to have osteoarthritis than men, and also suffer from more severe osteoarthritis. The prevalence of osteoarthritis varies among racial and ethnic groups. For example, across Europe, diagnosed osteoarthritis varies from 2.8% in Romania to 18.3% in Hungary, with a higher prevalence in females than in males. Several studies have indicated that osteoarthritis is inherited and account for between 50% and 65% of the genetic influences for hand, hip and knee osteoarthritis. Few congenital or developmental abnormalities have been associated with hip osteoarthritis later in life, and they only account for a small portion of the disease occurrence. Dietary factors are a subject of considerable interest in osteoarthritis since a deficiency of vitamins D, C and selenium is associated with increased risk of osteoarthritis.

Local risk factors related to osteoarthritis include obesity; injury/surgery; occupation; physical activity/sports; mechanical factors; alignment; and laxity. Obesity and being overweight have long been recognized as risk factors for osteoarthritis, especially osteoarthritis of the knee. The Framingham Study demonstrated that women who had lost about 5 kg had a significant reduction in the risk of development of symptomatic and radiographic knee osteoarthritis. Severe injury to the structures of the joint, particularly a trans-articular fracture, meniscal tear requiring meniscectomy, or anterior cruciate ligament injury, can result in an increased risk of osteoarthritis and musculoskeletal symptomatology. The risks of knee osteoarthritis associated with kneeling and squatting were much higher among subjects who were overweight or whose job also involved lifting. Studies examining the relationship between sports activities and subsequent osteoarthritis have produced conflicting results. Nevertheless, there is evidence that long distance runners and soccer players are at a higher risk for the development of knee osteoarthritis. It was demonstrated that abnormal anatomic alignment was strongly associated with accelerated structural knee deterioration under high compressive stress. Knee laxity is another risk factor for knee osteoarthritis. Varus-valgus knee laxity is greater in patients who have idiopathic disease than in the knees of control groups, suggesting that a portion of the increased laxity of knee osteoarthritis precedes disease development.

The main strategies for osteoarthritis management are to reduce pain and inflammation, slow cartilage degradation, improve function, reduce patient disability and improve the overall quality of life. Since no highly effective drugs exist, and surgical options are expensive and not widely accessible, prevention is a major strategy in osteoarthritis. Nevertheless, only a small number of interventions were identified as effective in osteoarthritis prevention, as follows: weight control; occupational injury prevention; sports injury prevention; improvement of misalignment (improper alignment) by orthotics or bracing.

Once osteoarthritis is diagnosed, by symptomatic signs or radiographic X-ray technology, the main goal is to minimize the complications of the disease.

Common therapeutic strategies for treating osteoarthritis include non-pharmacological treatments, such as, physiotherapy and occupational therapy; standard pharmacological treatments, such as, painkillers, anti-inflammation agents and drugs for topical applications; intra-articular treatments, such as, corticosteroids, hyaluronans, and tidal irrigation; and surgical arthroscopy, such as, osteomy, UKR (unicompartmental knee replacement) and total joint arthroplasty (knee or hip).

Orthopedic braces and orthoses are also applied for treating, or alleviating symptoms associated with, osteoarthritis. Orthopedic (orthotic) braces and orthoses are wearable devices which are used to improve the function of the musculoskeletal system. As such, these devices are typically used for rehabilitation from trauma and illness, mainly by trauma and arthritis patients.

Orthopedic braces and orthoses may be divided into groups according to the treated joint and intended treatment, as follows:

• Knee braces which include: knee braces for osteoarthritis and ligament injuries, and post-surgery knee braces.

• Foot and ankle braces which include soft braces and hinged braces.

• Upper extremity braces and orthoses which include wrist braces and hand orthoses, shoulder orthoses, and elbow braces.

• Neck and spine braces and orthoses.

• Exoskeletons.

According to the NIH National Institute on Aging (NIA), knees are among the joints most commonly affected by osteoarthritis. Symptoms of knee osteoarthritis include: stiffness; swelling; and pain in the knees, that can make it difficult to walk, climb, and get in and out of chairs and bathtubs. Osteoarthritis in the knees can lead to disability, and hence typically requires knee braces and support. Knee braces, orthotics, and appropriate footwear can reduce pain and improve function in people with poor alignment.

Provided herein are orthopedic braces for wearing by a subject in the vicinity of a joint, according to some embodiments. According to some embodiments, the orthopedic braces provide treatment of the joint.

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure. In the figures, like reference numerals refer to like parts throughout. Throughout the figures of the drawings, different superscripts for the same reference numerals are used to denote different embodiments of the same elements. Embodiments of the disclosed devices and systems may include any combination of different embodiments of the same elements. Specifically, any reference to an element without a superscript may refer to any alternative embodiment of the same element denoted with a superscript. Components having the same reference number followed by different lowercase letters may be collectively referred to by the reference number alone. If a particular set of components is being discussed, a reference number without a following lowercase letter may be used to refer to the corresponding component in the set being discussed.

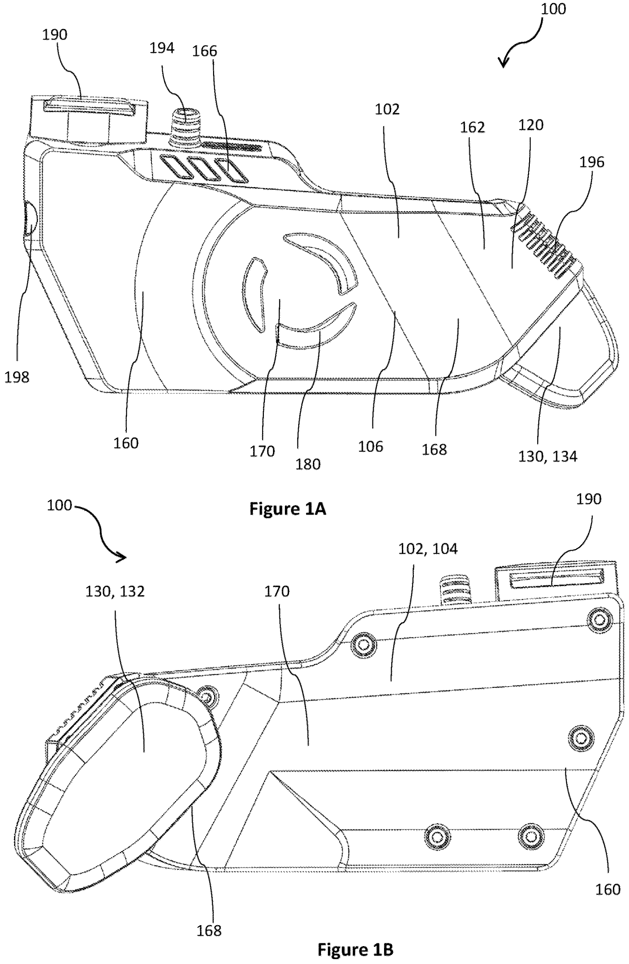

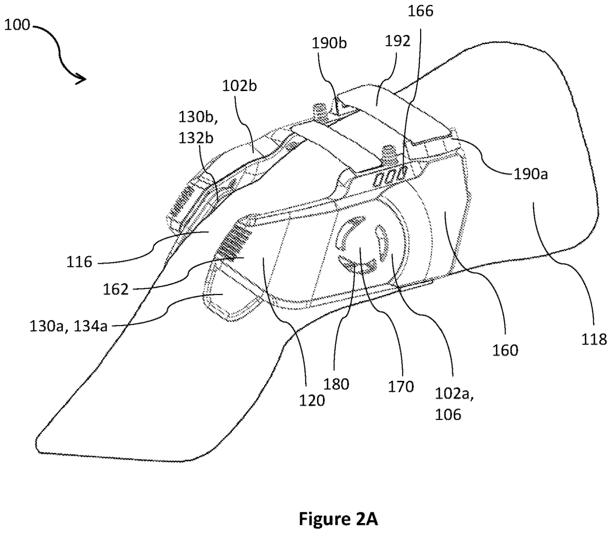

Reference is now made to Figures 1A-B, 2A-B, 3A-B, 4 and 5A-B. Figure 1A shows a view in perspective of an orthopedic brace 100 from an external viewpoint. Figure 1B shows a view in perspective of orthopedic brace 100 from an internal viewpoint. Figures 2A-B show views in perspective of different configurations of orthopedic brace 100 when worn on a subject's leg.

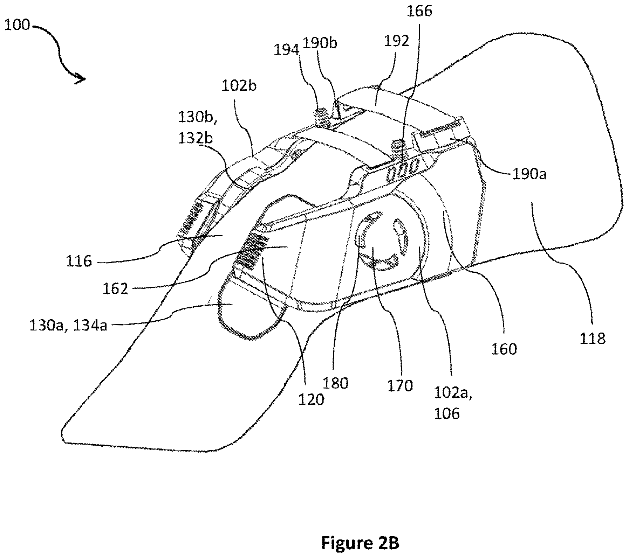

The term "internal" and "external", as well as derivatives thereof, such as "internally" and "externally" refer to sides as can be witnessed in Figures 2A and 2B. Specifically, the internal side is the side of orthopedic brace 100, or any device or element disclosed herein, which is closer to the subject's body when orthopedic brace 100 is worn. Accordingly, "internally" refers to the direction towards the subject's body. Similarly, the external side is the side of orthopedic brace 100, or any device or element disclosed herein, which is farther from the subject's body when orthopedic brace 100 is worn and "externally" refers to the direction away the subject's body. As seen in Figures 2A-B, orthopedic brace 100 may be constructed from 2 separate housings 102a and 102b as will be elaborated below, according to some embodiments. Figures 2A-B depict a situation that orthopedic brace 100 is worn by a subject and the internal face (104a and 104b) of each housing (102a and 102b respectively) is in contact with the subject's body, while the external face (106a and 106b) of each housing (102a and 102b respectively) does not contact the subject's body.

Figures 2A-B are described with respect to knee braces, in particular an active knee brace. However, it should be understood that the invention can be implemented for other joints, such as the elbow, wrist, ankle, shoulder, hip, spine, finger/toe joints, etc., *mutatis mutandis*. In addition, the presently presented design of orthopedic brace 100 in the figures is described for human subjects. However, orthopedic brace 100 may be designed and dimensioned to be worn on animal. According to some embodiments, the subject is a human subject.

According to some embodiments, the present invention provides orthopedic brace 100 comprising at least one housing 102, at least one substantially flat thermoelectric cooler (TEC) 120 and at least one heat transferring medium 130.

According to some embodiments, thermoelectric cooler 120 is disposed within housing 102, and thus it is hidden from view in Figures 1A-B and 2A-B. Nevertheless, its position is pointed out in these figures.

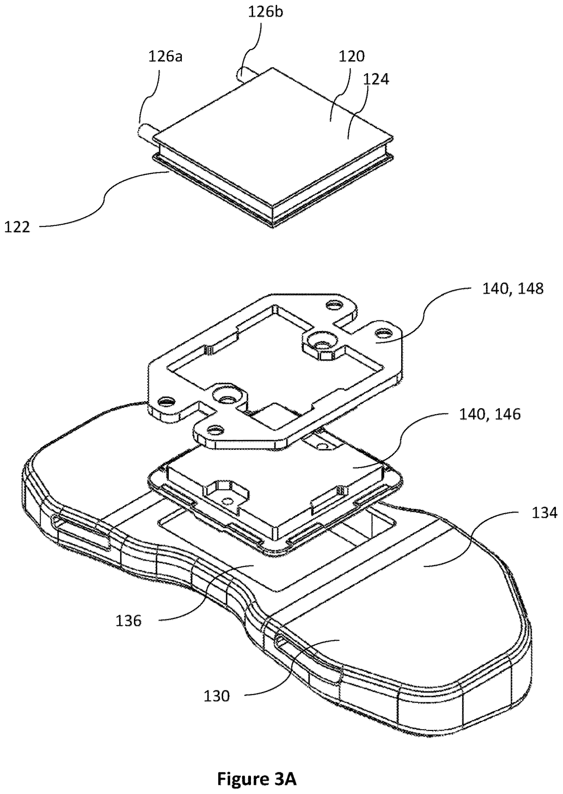

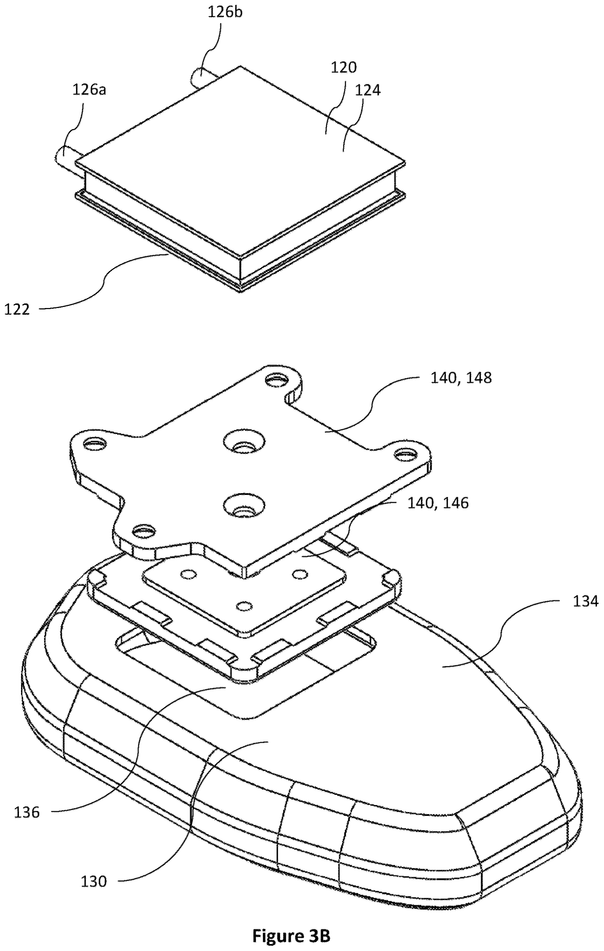

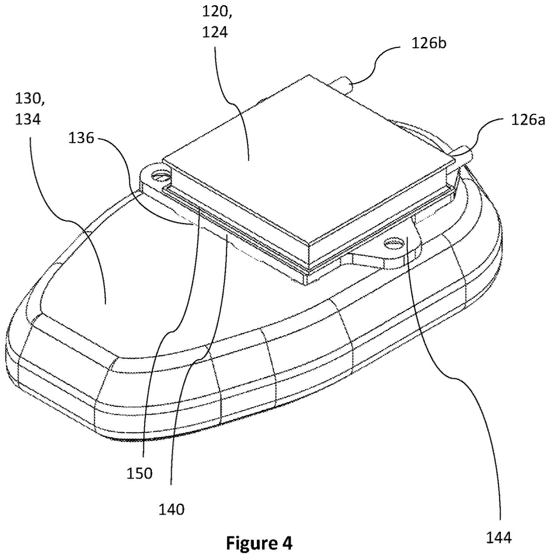

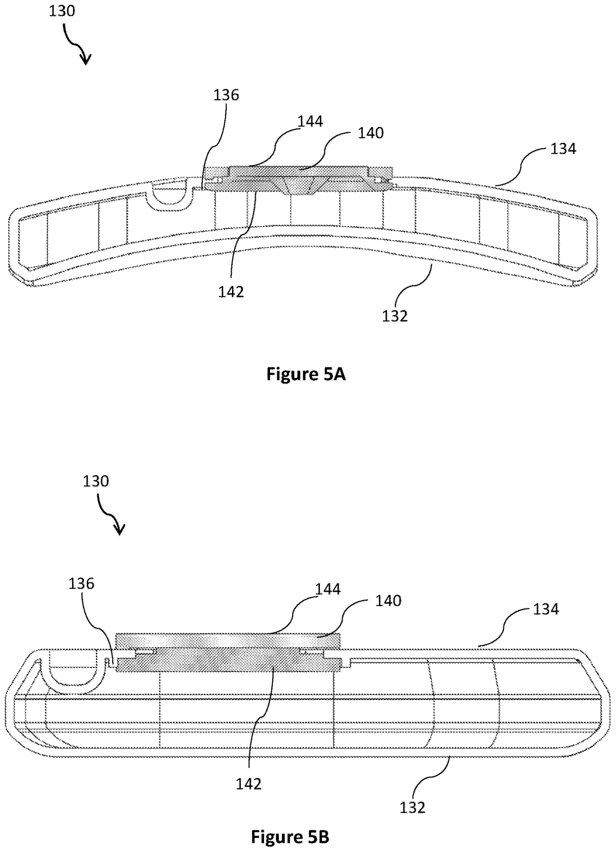

Figures 3A-B, 4 and 5A-B show assemblies of a thermoelectric cooler 120 and a heat transferring medium 130, according to some embodiments, in an exploded view (Figures 3A and 3B) and assembled (Figures 4 and 5A-B).

Thus, the structure of orthopedic brace 100 may be appreciated from the combination of Figures 1A-B, 2A-B, 3A-B, 4 and 5A-B.

Thus, according to some embodiments, there is provided an orthopedic brace 100 comprising at least one housing 102. Specifically, while Figures 1A and 1B, each shows orthopedic brace 100 with one housing 102, the configuration of orthopedic brace 100 with two housings 102, each from one side of the subject's knee, is portrayed in Figures 2A-B.

According to some embodiments, housing 102 has an internal housing face 104 and an external housing face 106.

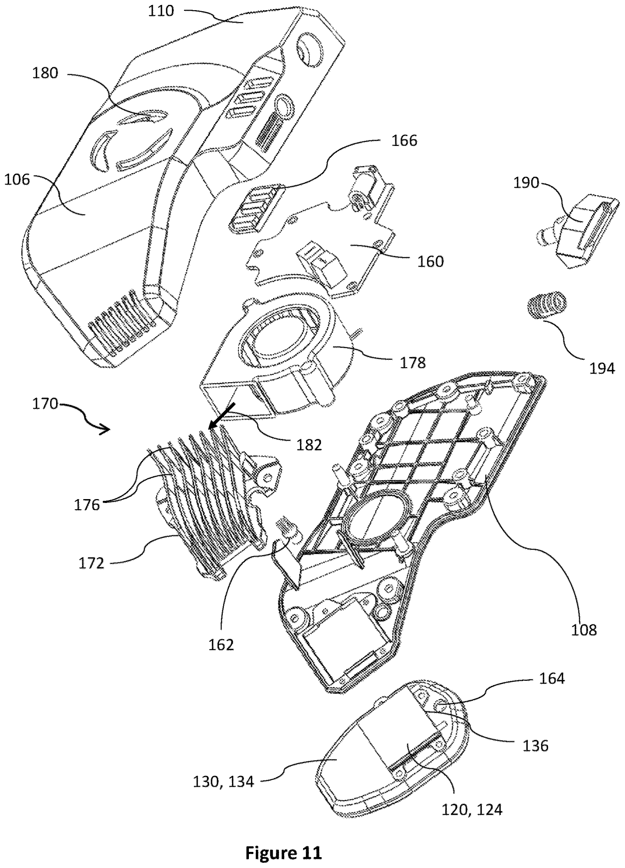

As can be appreciated from Figures 1A-B, 2A-B and 11 housing 102 is included in orthopedic brace 100 for housing various components of orthopedic brace 100. In Figure 11, housing 102 is shown as constructed from 2 parts - internal housing part 108, which is generally in contact or in close proximity with the subject's body part 118, when orthopedic brace 100 is worn, and external housing part 110, which is farther from the body part 118. Together, internal housing part 108 and external housing part 110 construct housing 102, with a hollow cavity inside for disposing different elements of orthopedic brace 100, according to some embodiments.

According to some embodiments, housing 102 has a rigid frame on or into which various components of the active orthopedic brace 100 are mounted, disposed, housed, held, coupled, connected or attached, as detailed herein.

The term "relatively rigid frame" as used herein refers to a structure or otherwise a scaffold that is sufficiently rigid to retain thereon the components of orthopedic brace 100 without undergoing substantial structural deformation. Yet, the rigidity of the frame is adjusted such that the contact between housing 102 of orthopedic brace 100 and the body part 118 of the subject on which it is mounted is pleasant.

In addition, and as detailed below, when referring to thermoelectric cooler 120 and the Peltier effect, thermoelectric cooler 120 has the capability to provide sharp and substantial temperature variations in short time, according to some embodiments. This characteristic, while being important to the implementation of orthopedic brace 100, according to some embodiments, may expose the subject to the danger of experiencing he sharp changes of temperatures and suffering from frostbite or burns.

Thus, according to some embodiments, housing 102 is made of a thermally insulating material. According to some embodiments, housing 102 is made of a material having thermal conductivity of no more than 2 $W*m^{-1}*K^{-1}$. According to some embodiments, housing 102 is made of a material having thermal conductivity of no more than 1.5 $W*m^{-1}*K^{-1}$. According to some embodiments, housing 102 is made of a material having thermal conductivity of no more than 1 $W*m^{-1}*K^{-1}$. According to some embodiments, housing 102 is made of a material having thermal conductivity of no more than 0.5 $W*m^{-1}*K^{-1}$. According to some embodiments, internal housing part 108 is made of a thermally insulating material. According to some embodiments, internal housing part 108 is made of a material having thermal conductivity of no more than 2 $W*m^{-1}*K^{-1}$. According to some embodiments, internal housing part 108 is made of a material having thermal conductivity of no more than 1.5 $W*m^{-1}*K^{-1}$. According to some embodiments, internal housing part 108 is made of a material having thermal conductivity of no more than 1 $W*m^{-1}*K^{-1}$. According to some embodiments, internal housing part 108 is made of a material having thermal conductivity of no more than 0.5 $W*m^{-1}*K^{-1}$. According to some embodiments, external housing part 110 is made of a thermally insulating material. According to some embodiments, external housing part 110 is made of a material having thermal conductivity of no more than 2 $W*m^{-1}*K^{-1}$. According to some embodiments, external housing part 110 is made of a material having thermal conductivity of no more than 1.5 $W*m^{-1}*K^{-1}$. According to some embodiments, external housing part 110 is made of a material having thermal conductivity of no more than 1 $W*m^{-1}*K^{-1}$. According to some embodiments, external housing part 110 is made of a material having thermal conductivity of no more than 0.5 $W*m^{-1}*K^{-1}$.

According to some embodiments, housing 102 is composed of a plastic polymer. According to some embodiments, internal housing part 108 is composed of a plastic polymer. According to some embodiments, external housing part 110 is composed of a plastic polymer. According to some embodiments, the plastic polymer is selected from the group consisting of polyamide, polycarbonate, polyethylene, polypropylene, polystyrene, polyurethane, polyvinyl chloride, polyvinylidene chloride, acrylonitrile butadiene styrene, polytetrafluoroethylene, phenolics (phenol formaldehyde), melamine formaldehyde, polyetheretherketone, polyetherimide, polyimide polymer, and combinations thereof. Each possibility represents a separate embodiment.

As specified above, according to some embodiments, housing 102 has an internal housing face 104 and an external housing face 106. It is to be understood that when housing 102 is composed of internal housing part 108 and external housing part 110, internal housing face 104 is a face of internal housing part 108 and external housing face 106 is a face of external housing part 110.

According to some embodiments, housing 102 is structured to be worn by a subject, in a vicinity of a subject's joint 116. According to some embodiments, internal housing face 104 is structured to be worn by a subject, in a vicinity of a subject's joint 116. The phrase "in the vicinity of a subject's joint" refers to close proximity to subject's joint 116 as shown in Figures 2A-B. Thus, it is to be understood that housing 102 and internal housing face 104 have structural features which enable wearing in the vicinity of subject's joint 116. According to some embodiments, at least a portion of orthopedic brace 100 is contacting a subject's body part 118 when orthopedic brace 100 is worn. It is also to be understood that subject's body part 118 is a body part of the subject, which is closest to subject's joint 116. Thus, the skilled person would appreciate said structural features enabling wearing in the vicinity of a human joint (in contrast e.g. with hat-shaped devices for wearing on a head). According to some embodiments, subject's joint 116 is selected from the group consisting of knee joint, an elbow joint, a wrist joint, an ankle joint, a shoulder joint, a hip joint, a spine joint, a finger joint, and a toe joint. Each possibility represents a separate embodiment. According to some embodiments, the joint is a knee joint. For example, it is to be understood that the configuration of orthopedic brace 100 with two housings 102, each from one side of the subject's knee, as portrayed in Figures 2A-B, is designed to be worn in the vicinity of a human knee. Modifications may provide designs for wearing e.g. in the vicinity of a human elbow joint.

Internal housing face 104 may directly or indirectly contact the subject's body part 118 when orthopedic brace 100 is worn, according to some embodiments. For example, a padding layer may be incorporated in orthopedic brace 100 internally to internal housing face 104, such that the padding layer is separating between housing 102 and the body part 118, and contributes to a better sensation and interaction of wearing orthopedic brace 100. According to some embodiments, the padding includes a fabric, such as cloth.

An important feature of the present orthopedic brace 100 is the temperature control provided to the subject's joint, which is facilitated by a thermoelectric cooler (TEC), specifically by thermoelectric cooler 120.

According to some embodiments, orthopedic brace 100 comprises thermoelectric cooler 120. According to some embodiments, thermoelectric cooler 120 is substantially flat.

As detailed herein thermoelectric cooler 120 is a flat electronic device, which upon application of electric current thereto, creates a temperature variation, whereby one of its flat sides is getting hot and the other is getting cold. This is due to the thermoelectric effect.

The thermoelectric effect is the direct conversion of temperature differences to electric voltage and *vice versa* via a thermocouple. A thermoelectric device creates a voltage when there is a different temperature on each side. Conversely, when a voltage is applied to it, heat is transferred from one side to the other, creating a temperature difference. At the atomic scale, an applied temperature gradient causes charge carriers in the material to diffuse from the hot side to the cold side.

This effect can be used to generate electricity, measure temperature or change the temperature of objects. Because the direction of heating and cooling is affected by the applied voltage, thermoelectric devices can be used as temperature controllers.

The term "thermoelectric effect" encompasses three interrelated identified effects: the Seebeck effect, Peltier effect, and Thomson effect.

The Peltier effect, discovered by Jean Peltier in 1834, is an important Thermoelectric Phenomenon that relates to the energy transfer (positive or negative) that occurs, over and above Joule Heating, at the junction of two dissimilar materials when an electric current pass through it. When the junction is maintained at a given temperature, the Peltier-effect results in the equivalent of a heat addition or heat removal the Peltier heat-which is reversible and is proportional to the current. Thus,

Peltier heat = πI, where π is the Peltier coefficient and I is the electric current. π depends on the materials forming the junction and the temperature.

The Peltier effect is one of the key phenomena (along with the Thompson effect) determining the electromagnetic force generated in a thermocouple used for temperature measurement. For a thermocouple of materials A and B, with one junction at a constant temperature and the other at (absolute) temperature T,

$$d\varepsilon_{AB}/dT = \pi_{AB}/T,$$

where $\varepsilon_{AB}$ is the thermocouple electromagnetic force generated at the junction of materials A and B. This equation can be used to calculate the Peltier coefficient for the combination of materials A and B.

Thermoelectric cooling uses the Peltier effect to create a heat flux at the junction of two different types of materials. A Peltier cooler, heater, or thermoelectric heat pump is a solid-state active heat pump which transfers heat from one side of the device to the other, with consumption of electrical energy, depending on the direction of the current. Such an instrument is also called a Peltier device, Peltier heat pump, solid state refrigerator, or thermoelectric cooler (TEC). It can be used either for heating or for cooling, although in practice the main application is cooling. It can also be used as a temperature controller that either heats or cools.

A Peltier cooler can also be used as a thermoelectric generator. When operated as a cooler, a voltage is applied across the device, and as a result, a difference in temperature will build up between the two sides. When operated as a generator, one side of the device is heated to a temperature greater than the other side, and as a result, a difference in voltage will build up between the two sides (the Seebeck effect).

According to some embodiments, thermoelectric cooler 120 is disposed at least partially within housing 102. According to some embodiments, thermoelectric cooler 120 is disposed within housing 102.

According to some embodiments, thermoelectric cooler 120 has an internal TEC face 122 and an external TEC face 124. According to some embodiments, external TEC face 124 is facing external housing face 106. According to some embodiments, internal TEC face 122 is facing internal housing face 104. According to some embodiments, internal TEC face 122 is facing the subject's joint 116, when orthopedic brace 100 is worn by the subject. According to some embodiments, internal TEC face 122 is facing the subject's body part 118, when orthopedic brace 100 is worn by the subject.

The term "facing" as used herein refers to a positional relation between two elements. Two elements, which face each other may or may not be connected and may or may not come in constant or temporary contact. Facing relations disclosed herein may further be appreciated from the figures.

According to some embodiments, upon application of electrical current to thermoelectric cooler 120, the temperature of external TEC face 124 is being either elevated or lowered and the temperature of internal TEC face 124 is being either lowered or elevated in the opposite direction. For example, electric current may be applied to thermoelectric cooler 120 at 25°C, which will elevate the temperature of external TEC face 124 to a temperature above 25°C and will lower the temperature of internal TEC face 122 to a temperature below 25°C. In another example, electric current may be applied to thermoelectric cooler 120 at 25°C, which will elevate the temperature of external internal TEC face 122 to a temperature above 25°C and will lower the temperature of internal external TEC face 124 to a temperature below 25°C. According to some embodiments, upon application of electrical current to thermoelectric cooler 120, the temperature of external TEC face 124 is being elevated and the temperature of internal TEC face 124 is being lowered. It is to be understood that the temperatures are lowered or elevated with respect to the temperature of an element before the application of electric current, specifically, with respect to the ambient temperature of the element.

According to some embodiments, the current applied to thermoelectric cooler 120 is a controlled current. According to some embodiments, controlling the current includes controlling the current magnitude and/or controlling the current direction. Generally, thermoelectric coolers behave differently upon application of current in a reverse direction. For example, upon application of current in a first direction one of the faces of the thermoelectric cooler gets hot and the other gets cold, while reversing the current direction also reverses the roles of the faces, wherein the formerly cold face heats and the formerly hot face gets hot. An optional electronic device configured to switch voltage polarity and thus current direction is an H-bridge.

An H-bridge will be detailed below as a component of control unit 160, when control unit 160 is detailed. Here are presented embodiments for an optional H-bridge, which may or may not be associated with control unit 160. According to some embodiments, orthopedic brace 100 comprises an H-bridge (not shown) disposed within housing 102. According to some embodiments, the H-bridge configured to switch the direction of electrical current applied to thermoelectric cooler 120 between a first direction and an opposite direction. According to some embodiments, the H-bridge is configured to switch the voltage polarity applied to thermoelectric cooler 120. According to some embodiments, upon application of electrical current in the first direction to thermoelectric cooler 120, the temperature of external TEC face 124 is being elevated and the temperature of internal TEC face 122 is being lowered. According to some embodiments, upon application of electrical current in the opposite direction to thermoelectric cooler 120, the temperature of external TEC face 124 is being lowered and the temperature of internal TEC face 122 is being elevated.

According to some embodiments, thermoelectric cooler 120 has power consumption in the range of 1 to 120 Watts. According to some embodiments, thermoelectric cooler 120 has power consumption in the range of 10 to 30 Watts. According to some embodiments, thermoelectric cooler 120 has power consumption in the range of 12 to 24 Watts.

It is further to be appreciated from the disclosed herein that thermoelectric cooler 120 functions to directly or indirectly heat or cool other elements of orthopedic brace 100 (e.g. heat transferring connector 140 and/or heat transferring medium 130), according to some embodiments. For efficient heat conductance smooth surfaces of internal TEC face 122 and/or external TEC face 124 may be provided.

According to some embodiments, TEC face 122 has a smooth surface. According to some embodiments, external TEC face 124 has a smooth surface. According to some embodiments, each one of internal TEC face 122 and external TEC face 124 has a smooth surface.

The term "smooth" as used in this application may be understood to include, but is not limited to, any structure(s) or functionality having a generally even surface, which may be generally free from projections or indentations. Smooth surfaces have low surface roughness values.

According to some embodiments, the smooth surface of TEC face 122 has an average surface roughness of no more than 50, 40, 30, 20, 10, 5, 4, 3, 2, 1, or 0.5 micrometers. According to some embodiments, the smooth surface of external TEC face 124 has an average surface roughness of no more than 50, 40, 30, 20, 10, 5, 4, 3, 2, 1, or 0.5 micrometers.

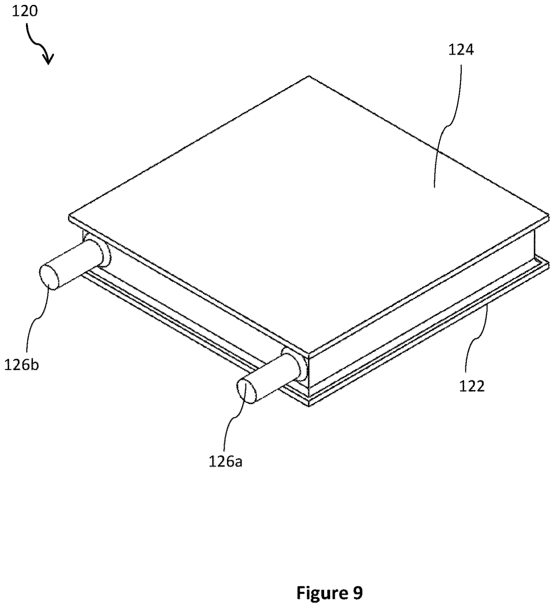

With reference to Figure 9, which shows thermoelectric cooler 120 from an external perspective view, it is seen that thermoelectric cooler 120 is connected to 2 electric wires - 126a and 126b, according to some embodiments. The 2- wire configuration may help to control the current direction via voltage polarity as described herein.

According to some embodiments, thermoelectric cooler 120 comprises TEC electric wires 126 extending therefrom. According to some embodiments, TEC electric wires 126 are connected to a power source (not shown), and configured to deliver electrical current to thermoelectric cooler 120. According to some embodiments, TEC electric wires 126 are connected to a power source through control unit 160, as detailed below, and configured to deliver electrical current to thermoelectric cooler 120. According to some embodiments, the electrical current applied to thermoelectric cooler 120 is controlled as detailed herein with respect to the current direction and magnitude.

Operational temperatures of thermoelectric cooler 120 are typically in the range of about -40°C to about 80°C. According to some embodiments, the operational temperature of thermoelectric cooler 120 is in the range of about -20°C to about 80°C. According to some embodiments, thermoelectric cooler 120 is operated in the temperature range of about -5°C to about 70°C. According to some embodiments, thermoelectric cooler 120 is operated at a temperature range of 0°C to about 50°C.

Reference is made back to Figures 1A-B, 2A-B, 3A-B, and 4, as well as to Figures 5A-B, 6A-B, 7A-B and 8 According to some embodiments, orthopedic brace 100 further comprises at least one heat transferring medium 130. According to some embodiments, heat transferring medium 130 is coupled to thermoelectric cooler 120. According to some embodiments, heat transferring medium 130 is indirectly connected to thermoelectric cooler 120. According to some embodiments, heat transferring medium 130 is directly connected to thermoelectric cooler 120. According to some embodiments, heat transferring medium 130 is directly or indirectly connected to thermoelectric cooler 120. According to some embodiments, heat transferring medium 130 is thermally coupled to thermoelectric cooler 120.

As used herein, the term "directly connected" refers to a configuration wherein elements are attached to each other without any intermediate elements therebetween, except for any means of attachment (e.g. adhesive, such as first thermal paste 150 and second thermal paste 152).

As used herein, the term "indirectly connected" refers to a configuration wherein elements are attached to each other with one or more intermediate elements therebetween.

The term "coupled", as used herein, is a relation between two elements, which influence one another. Unless specified otherwise, the influence is mechanical, meaning that application of one of the elements has a mechanical influence over the other element. Two coupled elements may be connected, although not necessarily directly, and not necessarily mechanically. When not mechanical coupling relations are referred herein, it will be specified, e.g. thermal coupling between heat conducting elements or operational coupling of a processing unit with an electronic device.

The term "thermally coupled" is defined as having as two elements or materials in relative configuration such that heat is effectively exchanged (conducted) between the two elements or materials.

Generally, and as further detailed below, heat transferring medium 130 is configured to exchange heat between thermoelectric cooler 120 and subject's joint 116, when orthopedic brace 100 is operated. In other words, when internal TEC face 122 is cold, heat transferring medium 130 is transferring the cold temperature to subject's joint 116, and when internal TEC face 122 is hot, heat transferring medium 130 is transferring the hot temperature to subject's joint 116.

It can be appreciated from Figure 3A-B and 4 that they portray a situation in which heat transferring medium 130 is indirectly connected to thermoelectric cooler 120 and thermally coupled to thermoelectric cooler 120. Specifically, in the option described by said figures, an intermediate heat transferring connector 140 is connecting between heat transferring medium 130 and thermoelectric cooler 120. This option is elaborated herein in detail. However, heat transferring medium 130 may come in direct contact with thermoelectric cooler 120, and absorb the temperature therefrom to be transferred to the subject's joint 116, according to some embodiments. For example, heat transferring medium 130 may be a thermally conductive alloy or metal position to contact both thermoelectric cooler 120 and the subject's body part 118 in the vicinity of the subject's joint 116, such that the temperature is conducted efficiently, according to some embodiments.

According to some embodiments, heat transferring medium 130 comprises a first thermally conductive material, as detailed below.

According to some embodiments, heat transferring medium 130 has a heat transferring medium external face 134 and a heat transferring medium internal face 132.

According to some embodiments, heat transferring medium external face 134 is facing external housing face 106. According to some embodiments, heat transferring medium external face 134 is facing internal housing face 104. According to some embodiments, heat transferring medium external face 134 is contacting internal housing face 104. According to some embodiments, heat transferring medium external face 134 is connected to internal housing face 104. According to some embodiments, heat transferring medium external face 134 is connected to housing 102. According to some embodiments, heat transferring medium external face 134 is facing thermoelectric cooler 120. According to some embodiments, heat transferring medium internal face 132 is facing the subject's body part 118, when orthopedic brace 100 is worn by the subject. According to some embodiments, heat transferring medium internal face 132 is facing the subject's joint 116, when orthopedic brace 100 is worn by the subject.

According to some embodiments, heat transferring medium external face 134 is thermally coupled to thermoelectric cooler 120. According to some embodiments, heat transferring medium internal face 132 is thermally coupled to the subject's body part 118, when orthopedic brace 100 is worn by the subject. According to some embodiments, heat transferring medium internal face 132 is thermally coupled to the subject's joint 116, when orthopedic brace 100 is worn by the subject.

According to some embodiments, upon application of electrical current to thermoelectric cooler 120, the temperature of external TEC face 124 is being either elevated or lowered and the temperature of internal TEC face 124 is being either lowered or elevated in the opposite direction, thereby lowering or elevating the temperature of heat transferring medium 130. For example, electric current may be applied to thermoelectric cooler 120 at 25°C, which will elevate the temperature of external TEC face 124 to a temperature above 25°C and will lower the temperature of internal TEC face 122 to a temperature below 25°C. This will also lower the temperature of heat transferring medium 130 through heat conductance between heat transferring medium 130 and thermoelectric cooler 120. Said temperature elevation will cool the subject's joint 116, according to some embodiments. In another example, electric current may be applied to thermoelectric cooler 120 at 25°C, which will elevate the temperature of external internal TEC face 122 to a temperature above 25°C and will lower the temperature of internal external TEC face 124 to a temperature below 25°C. This will also elevate the temperature of heat transferring medium 130 through heat conductance between heat transferring medium 130 and thermoelectric cooler 120. Said temperature elevation will heat the subject's joint 116, according to some embodiments.

According to some embodiments, the first thermally conductive material is selected from the group consisting of a thermally conductive gel, thermally conductive solid, a thermally conductive liquid, a thermally conductive solution, a thermally conductive emulsion and a thermally conductive suspension. Each option represents a separate embodiment.

According to some embodiments, the first thermally conductive material is a thermally conductive semi solid. According to some embodiments, the first thermally conductive material is a thermally conductive semi solid at room temperature.

Specifically, according to some embodiments, the first thermally conductive material and/or heat transferring medium 130 may be soft and at least semi-fluid at room temperature, while upon cooling, they may solidify. Such configuration may be beneficial for fixing the body part 118 of the subject at a conformation, which is preferable as part of the joint treatment.

According to some embodiments, lowering the temperature of heat transferring medium 130 comprises setting the temperature of heat transferring medium 130 at a first temperature. According to some embodiments, the first temperature is lower than ambient temperature. According to some embodiments, at the first temperature the thermally conductive material is harder than it is at ambient temperature. According to some embodiments, heat transferring medium 130 is substantially rigid at the first temperature. According to some embodiments, heat transferring medium 130 is substantially solid at the first temperature. According to some embodiments, heat transferring medium 130 is in a non-bendable state when in the first temperature. According to some embodiments, heat transferring medium 130 undergoes a phase change upon cooling from ambient temperature to the first temperature. It is to be understood that the rigidity/ flexibility/ bendability of heat transferring medium 130 may not be change dramatically instantly, and its transition from bendable to non-bendable state and *vice-versa*, may require several minutes.

The terms "bendable" and "non-bendable" are defined with respect to application of force by a standard human limb. If a force applied by a standard limb can contort an object by more than 10°, it will be considered bendable, while if said force is not able to bend such an object, the object will be considered non-bendable.

According to some embodiments, heat transferring medium 130 is in a bendable state when in room temperature. According to some embodiments, heat transferring medium 130 is substantially flexible at room temperature. According to some embodiments, heat transferring medium 130 is substantially pliable at the first temperature. According to some embodiments, upon application of electrical current to thermoelectric cooler 120 the temperature of heat transferring medium 130 is either lowered or elevated, thereby heat transferring medium 130 being in either the non-bendable state or the bendable state respectively. According to some embodiments, thermoelectric cooler 120 is configured to cool heat transferring medium 130 thereby to transform it from the bendable state to the non-bendable state.

According to some embodiments, when orthopedic brace 100 is worn by the subject and when in heat transferring medium 130 is in a non-bendable state, subject's joint 116 is substantially fixed in a predetermined conformation. According to some embodiments, when orthopedic brace 100 is worn by the subject and when in heat transferring medium 130 is in a bendable state, subject's joint 116 is substantially movable.

According to an exemplary embodiment, the first thermally conductive material comprises diethyl sulfoxide, having a melting point or conversion temperature of 14°C. Thus, at a temperature above 14°C, the first thermally conductive material is in a liquid state, and as such it is flexible and enables free movement of the joint, on which orthopedic brace 100 is placed. Cooling the first thermally conductive material to a temperature lower than 14°C, converts the first thermally conductive material to its solid state, resulting with fixing the subject's joint 116 in a stable state.

As such, the design of orthopedic brace 100 disclosed herein in configured such that heat transferring medium 130 can perform two functions: (i) thermal function - heating and cooling; and (ii) mechanical function - fixing the joint in a desired position, namely, stabilizing the joint.

According to another exemplary embodiment, the first thermally conductive material comprises polyethylene glycol PEG 400, having a conversion temperature of 4-8°C. Thus, at a temperature above 8°C, the first thermally conductive material is present in a liquid state, and as such it is flexible. Cooling the first thermally conductive material to a temperature lower than 4 °C, converts the first thermally conductive material to its solid state.

According to some embodiments, the first thermally conductive material includes a polymeric material. According to some embodiments, the first thermally conductive material comprises a silicone-based polymer, organic polymer, organic solvent, water-based solvent, or combinations thereof. In some embodiments, the first thermally conductive material comprises silicone based polymers having the formula $[R_2SiO]n$, where R is an alkyl group (e.g. methyl, ethyl), or a phenyl group, or a combination thereof. According to some embodiments, the silicone-based polymer of first thermally conductive material is silicon oil and/or silicon grease. In some embodiments, the silicone-based polymer of first thermally conductive material is polydimethylsiloxane (PDMS). According to some embodiments, the first thermally conductive material comprises an organic polymer such as polyethylene glycol (PEG) or polypropylene glycol (PPG), or a combination thereof. In some embodiments, the first thermally conductive material comprises an organic solvent, such as dimethyl sulfoxide or diethyl sulfoxide or a combination thereof. In other embodiments, the first thermally conductive material may include a combination of a silicone-based polymer, organic polymer, organic solvent and water.

According to some embodiments, a material with an efficient thermal conductivity may be added to the first thermally conductive material to improve its response to the temperature change. Such material may be metal, metal alloy, graphite and/or graphene.

According to some embodiments, the thermally conductive material is a thermally conductive gel. According to some embodiments, heat transferring medium 130 is a gel bag 130. It is to be understood that a "gel bag" is a packing of gel material within a bag. The bag itself typically include a polymeric material, e.g. a plastic bag. The bag material should be impermeable to the gel.

The gel bag includes a packing, which is made of flexible material. According to some embodiments, the packing is non-permeable to the gel composition contained therein. According to some embodiments, the bag may include, or be made of, flexible non-permeable material, such as, rubber, silicone, plastic polymer, or combinations thereof. According to some embodiments, the bag may include, or be made of, thermally conductive silicon rubber. According to some embodiments, the plastic polymer may include polyamide, polyethylene, polypropylene, polyvinyl chloride, ethylene vinyl acetate, co-polyester ether polymer, or combinations thereof.

In order to achieve quick response of heat transferring medium 130 to the temperature variation, it may be constructed relatively thin, as can be appreciated by the skilled in the art.

According to some embodiments, gel bag 130 has a heat transferring medium depth 1307 , measured between gel bag external face 134 and gel bag internal face 132. According to some embodiments, each of gel bag external face 134 and gel bag internal face 132 has a substantially equal area having a heat transferring medium length 1308 and a heat transferring medium width 1309. According to some embodiments, each of the heat transferring medium length 1308 and a heat transferring medium width 1309 is at least 2.5 times greater than heat transferring medium depth 1307. According to some embodiments, the heat transferring medium depth 1307 is in the range of 2 mm to 20 mm. According to some embodiments, each of the gel bag 130 external face area and the gel bag 130 internal face area has heat transferring medium length 1308 and heat transferring medium width 1309, each in the range of 10 mm to 250 mm.

According to some embodiments, heat transferring medium 130 is coupled to housing 102. According to some embodiments, heat transferring medium 130 is connected to housing 102. According to some embodiments, heat transferring medium 130 is connected to internal housing face 104. In an exemplary configuration shown in Figure 1B, heat transferring medium 130 is in contact and connected to housing 102 at internal housing face 104.

According to some embodiments, heat transferring medium 130 is at least partially disposed within housing 102.

According to some embodiments, the first thermally conductive material has a thermal conductivity in the range of 0.5 to 24,000 $W*m^{-1}*K^{-1}$. According to some embodiments, first thermally conductive material has a thermal conductivity of at least 1.5 $W*m^{-1}*K^{-1}$.

Reference now is made to heat transferring connector 140, which is hidden from view in Figures 1A-B and 2A-B, but may be seen in Figures 3A-B, 4 and 5A-B.

In general, heat transferring connector 140 is positioned between thermoelectric cooler 120 and heat transferring medium 130, as seen in Figures 3A-B, 4 and 5A-B, and functions as a mediator transferring heat between said elements, according to some embodiments.

According to some embodiments, orthopedic brace 100 further comprises a substantially flat heat transferring connector 140. According to some embodiments, heat transferring connector 140 is made of a second thermally conductive material.

According to some embodiments, heat transferring connector 140 has a heat transferring connector external face 144 coupled to thermoelectric cooler 120. According to some embodiments, heat transferring connector external face 144 is thermally coupled to thermoelectric cooler 120. According to some embodiments, heat transferring connector external face 144 is contacting thermoelectric cooler 120. According to some embodiments, heat transferring connector external face 144 is contacting internal TEC face 122. According to some embodiments, heat transferring connector external face 144 is connected to thermoelectric cooler 120. According to some embodiments, heat transferring connector external face 144 is connected to internal TEC face 122. According to some embodiments, heat transferring connector external face 144 is connected to thermoelectric cooler 120 by a first thermal paste 150. According to some embodiments, heat transferring connector external face 144 is connected to internal TEC face 122 by a first thermal paste 150. First thermal paste 150 is generally configured to allow attachment of thermoelectric cooler 120 through internal TEC face 122 with heat transferring connector 140 through heat transferring connector external face 144, while enabling efficient heat conductance between thermoelectric cooler 120 and heat transferring connector 140.

The term "thermal paste" refers to any suitable heat transferring agent that fills gaps that naturally occur when two flat surfaces attached. Thermal pastes are generally thermally conductive chemical compounds, which are commonly used as interfaces between heat sinks and heat sources such as high-power semiconductor devices. The main role of thermal paste is to eliminate air gaps or spaces (which act as thermal insulation) from the interface area in order to maximize heat transfer and dissipation.

According to some embodiments, thermal paste 150 is a thermally conductive chemical compound, composed of mixture of thermally conductive materials in the form of paste, gel and liquid. According to some embodiments, thermal paste 150 is composed of at least one polymerizable liquid compound based on epoxides, silicones, urethanes, and/or acrylates, added to at least one metal oxide such as aluminum oxide and/or zinc oxide and/or metal nitride such as aluminum nitride and/or boron nitride. According to some embodiments, first thermal paste 150 may be a combination of one of the compositions described herein. According to some embodiments, thermal paste 150 composed of at least one liquid metal, at least one fusible metal and at least one fusible metal alloy.

According to some embodiments, the fusible metal or alloy includes Mercury (Hg), mercury alloy, alkali metal, alkali metal alloy, gallium, gallium alloy, Bismuth (Bi), bismuth alloy, lead (Pb), lead alloy, tin (Sn) alloy, cadmium alloy, zinc alloy, indium (In) alloy, thallium alloy, or a combination thereof.

According to some embodiments, the mercury alloy is a mercury-thallium (Tl) or mercury-cesium alloy.

According to some embodiments, the alkali metal or alloy is selected from potassium, sodium, cesium, rubidium, francium, potassium-sodium, cesium-potassium, rubidium-potassium, cesium-potassium-sodium, and rubidium-potassium-sodium alloy.

According to some embodiments, the Gallium alloy is selected from gallium-indium, gallium-indium-tin, gallium-indium-zinc, and gallium-indium-tin-copper alloy.

According to some embodiments, the Bismuth alloy is selected from bismuth-indium, bismuth-tin, bismuth-lead, bismuth-indium-tin, bismuth-lead-tin, bismuth-lead-indium-tin, bismuth-lead-tin-cadmium, bismuth-lead-indium-tin-cadmium, and bismuth-lead-indium-tin-cadmium-thallium alloy.

According to some embodiments the lead alloy is lead-tin.

According to some embodiments, heat transferring connector 140 has a heat transferring connector internal face 142 facing heat transferring medium 130. According to some embodiments, heat transferring connector internal face 142 is contacting heat transferring medium 130. According to some embodiments, heat transferring connector internal face 142 is connected to heat transferring medium 130. According to some embodiments, heat transferring connector 140 is connected to heat transferring medium 130. According to some embodiments, heat transferring connector 140 thermally coupled to heat transferring medium 130.

According to some embodiments, heat transferring medium 130 comprises a heat transferring medium niche 136. According to some embodiments, heat transferring medium niche 136 is located at heat transferring medium external face 134. According to some embodiments, heat transferring medium niche 136 is penetrating from heat transferring medium external face 134 in the internal direction into heat transferring medium 130. According to some embodiments, heat transferring medium niche 136 is configured to accommodate at least a portion of heat transferring connector 140. According to some embodiments, heat transferring medium niche 136 is configured to accommodate a portion of heat transferring connector 140. According to some embodiments, heat transferring medium niche 136 is dimensioned to accommodate a portion of heat transferring connector 140.

Specifically, it is contemplated that penetration of the source of heat/cold into heat transferring medium 130 will allow efficient and more rapid temperature conductance to heat transferring medium 130 and to the user's body part 118 thereby. In addition, heat transferring medium niche 136 may help to fix and/or connect heat transferring medium 130 to the rest of orthopedic brace 100.

According to some embodiments, heat transferring connector 140 is flat. According to some embodiments, heat transferring connector 140 is made of a second thermally conductive material.

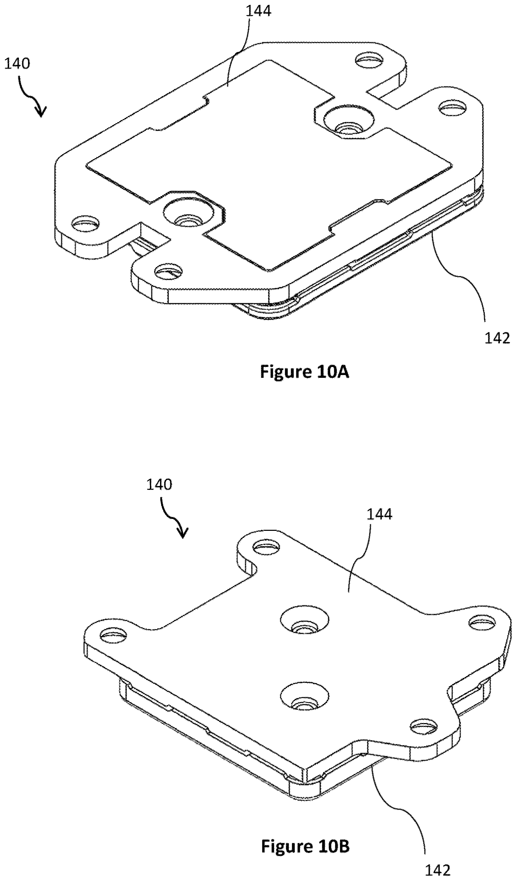

In Figures 3A and 10A, and in Figures 3B and 10B, heat transferring connector 140 is shown a made of two separate parts (146 and 148). Although such design is currently displayed, it is not obligatory for proper function of orthopedic brace 100, but it is currently contemplated for convenience. According to some embodiments, heat transferring connector 140 comprises heat transferring connector first part 146 and heat transferring connector second part 148.

In Figures 3A and 10A, heat transferring connector first part 146 is positioned to be in direct contact with internal TEC face 122. In Figure 3A, heat transferring connector first part 146 is thermally coupled to heat transferring medium 130, and thus to the subject body part 118, according to some embodiments. According to some embodiments, as seen in Figure 3A, heat transferring connector first part 146 is located within heat transferring medium niche 136. Further in Figure 3A heat transferring connector second part 148 is configured to fix heat transferring connector first part 146 within heat transferring medium niche 136, according to some embodiments. Heat transferring connector first part 146 may also be used to connect between thermoelectric cooler 120 and heat transferring medium 130, according to some embodiments, as show in Figure 3A. Thus, in the configuration of Figures 3A and 10A, heat transferring connector first part 146 is required to be a heat conductor and heat transferring connector second part 148 may be a conductor or insulator of heat.

In Figures 3B and 10B, each one of heat transferring connector first part 146 and heat transferring connector second part 148 are made of heat conducting material, according to some embodiments. In this configuration, thermoelectric cooler 120 is contacting and thermally coupled to heat transferring connector second part 148; heat transferring connector second part 148 is contacting and thermally coupled to heat transferring connector first part 146; and heat transferring connector first part 146 is contacting and thermally coupled to heat transferring medium 130, such that heat is conducted from thermoelectric cooler 120 to heat transferring medium 130. As in Figure 3A, also in the configuration of Figure 3B heat transferring connector second part 148 is configured to fix heat transferring connector first part 146 within heat transferring medium niche 136, according to some embodiments. Heat transferring connector first part 146 may also be used to connect between thermoelectric cooler 120 and heat transferring medium 130, according to some embodiments, as show in Figure 3B.

According to some embodiments, the second thermally conductive material comprises a metal, a metal alloy or a combination thereof. According to some embodiments, the second thermally conductive material comprises a metal.

According to some embodiments, the second thermally conductive material has a thermal conductivity in the range of 7 to 24,000 $W*m^{-1}*K^{-1}$. According to some embodiments, the second thermally conductive material has a thermal conductivity of at least 7.8 $W*m^{-1}*K^{-1}$.

According to some embodiments, the metal or metal alloy is selected from the group consisting of Aluminum (Al), Beryllium (Be), Bismuth (Bi), Chromium (Cr), Cobalt (Co), Copper (Cu), Gallium (Ga), Gold (Au), Indium (In), Iron (Fe), Lead (Pb), Magnesium (Mg), Mercury (Hg), Nickel (Ni), Potassium (K), Rare Earths, Rhodium (Rh), Samarium (Sm), Scandium (Sc), Silver (Ag), Sodium (Na), Titanium (Ti), Tin (Sn), Zinc (Zn), and combinations thereof.

According to some embodiments, the Aluminum alloy is selected from the group consisting of aluminum, aluminum-lithium, aluminum-copper, aluminum-Scandium, aluminum-magnesium, aluminum-titanium, beryllium-aluminum, aluminum-gallium, aluminum-magnesium-manganese, aluminum-nickel-cobalt, aluminum-copper-iron-nickel, and aluminum-copper-nickel-magnesium alloy.

According to some embodiments, the Chromium alloy is a chromium-nickel or chromium-iron alloy.

According to some embodiments, the Cobalt alloy is a cobalt-chromium-molybdenum, cobalt-chromium-tungsten-carbon, cobalt-tungsten-molybdenum-carbon, cobalt-chromium-nickel-iron-molybdenum-tungsten, or cobalt-chromium-molybdenum alloy.

According to some embodiments, the copper alloy is a copper-zinc, copper-tin, copper-nickel, copper-aluminum, copper-gallium, copper-tungsten, copper-silver, copper-gold, copper-lead, cooper-beryllium, copper-silver-gold, copper-tin-zinc, cooper-beryllium-iron, copper-nickel-iron, copper-nickel-manganese copper-aluminum-zinc, copper-indium-gallium, or copper-aluminum-zinc-tin alloy.

According to some embodiments, the gold alloy is a gold-copper, gold-silver, gold-rhodium, gold-iron, gold-silver-copper, gold-iron-copper, or gold-nickel-palladium alloy.

According to some embodiments, the iron alloy is an iron-carbon (carbon steel), iron-carbon-molybdenum, manganese, chromium or nickel (low alloy steel), iron-carbon-chromium (stainless steel), iron-carbon-chromium-nickel, iron-carbon-tungsten, iron-carbon-cobalt, iron-carbon-tungsten-cobalt, iron-carbon-manganese, or iron-gallium alloy.

According to some embodiments, the nickel alloy is a nickel-chromium, nickel-iron, nickel-carbon, nickel-silicon, nickel-molybdenum, nickel-aluminum, nickel-copper, nickel-titanium, nickel-cobalt, nickel-gallium, nickel-aluminum-cobalt, nickel-chromium-iron, nickel-copper-zinc, nickel-chromium-iron, nickel-iron-molybdenum, nickel-titanium-aluminum, nickel-copper-zinc-manganese, nickel-copper-iron-manganese, nickel-chromium-molybdenum-tungsten, nickel-chromium-silicon-magnesium, or nickel-chromium-cobalt-titanium alloy.

According to some embodiments, the silver alloy is a silver-copper, silver-gold, silver-platinum, silver-copper-gold, or silver-copper-germanium alloy.

According to some embodiments, the titanium alloy is a titanium-aluminum, titanium-gold, titanium-vanadium, titanium-aluminum-vanadium, or titanium-vanadium-chromium alloy.

According to some embodiments, heat transferring connector external face 144 is connected to internal TEC face 122 through a first thermal paste 150 disposed there between. It was found by the inventors of the present invention that inclusion of a thermal paste improves the heat conductivity between thermoelectric cooler 120 and heat transferring connector 140.

According to some embodiments, first thermal paste 150 has a thermal conductivity in the range of 0.5 to 100 $W*m^{-1}*K^{-1}$.

According to some embodiments, upon application of electrical current to thermoelectric cooler 120, the temperature of internal TEC face 122 is being either lowered or elevated, thereby lowering or elevating the temperature of heat transferring connector 140 respectively. According to some embodiments, upon the temperature of heat transferring connector 140 being either lowered or elevated, heat is conducted between heat transferring connector 140 and heat transferring medium 130 to lower or elevate the temperature of heat transferring medium 130 respectively. According to some embodiments, upon application of electrical current to thermoelectric cooler 120, the temperature of internal TEC face 122 is being either lowered or elevated, thereby lowering or elevating the temperature of heat transferring connector 140 respectively, and upon the temperature of heat transferring connector 140 being either lowered or elevated, heat is conducted between heat transferring connector and heat transferring medium 130 to lower or elevate the temperature of heat transferring medium 130 respectively.

Reference will now be made to control unit 160, which, according to some embodiments, may control various aspects of the operation and function of orthopedic brace 100. Control unit 160 is shown in Figure 11 and is hidden from view in Figures 1A-B and 2A-B by housing 102. The position of control unit 160, however, is less important than is function, which is detailed below.

According to some embodiments, orthopedic brace 100 further comprises a control unit 160. According to some embodiments, control unit 160 is electrically coupled to thermoelectric cooler 120.

The term "electrically coupled" is understood by the skilled in the art and refers to a functional and/or operational relation between two electronic components. This relation is conventional between processing units and electronic elements controlled thereby. In general an electronic component is electrically coupled to another electronic component can receive and/or send electronic signals therefrom or thereto, typically via electric wires.

According to some embodiments, control unit 160 is configured to control electrical current applied to thermoelectric cooler 120. According to some embodiments, control unit 160 is configured to determine electrical current applied to thermoelectric cooler 120. According to some embodiments, control unit 160 is configured to monitor electrical current applied to thermoelectric cooler 120.

According to some embodiments, control unit 160 is electrically coupled to a power source (not shown). The power source may be a power source external to orthopedic brace 100, according to some embodiments. According to some embodiments, control unit 160 is electrically coupled to an external power source through an external power connector inlet 198, as shown in Figure 1A. external power sources may be, but not limited to an external battery or a home electric system. The power source may be an internal power source, such as a battery within housing 102 (not shown).

According to some embodiments, control unit 160 is disposed within housing 102. According to some embodiments, control unit 160 is not in contact with any one or more of thermoelectric cooler 120, heat transferring medium 130 and heat transferring connector 140.

Control unit 160 may be, for example, a controller, such as a mini/micro controller (e.g. STM32 controller) with a control software running thereon. The control software may include algorithms to control thermoelectric cooler 120 as detailed below and/or other electronic components of orthopedic brace 100 and to create operation plans/treatment programs, monitoring points, heating/cooling time cycles, intervals or delays, cycle temperatures, etc. other electronic component, which may be controlled by control unit 160 include LED lamp 166, vibrating unit 168, fan 178 and temperature sensor 162.

According to some embodiments, control unit 160 comprises an H-bridge (not shown) configured to switch the direction of electrical current applied to thermoelectric cooler 120 between a first direction and an opposite direction. According to some embodiments, the H-bridge is configured to switch the voltage polarity applied to thermoelectric cooler 120. According to some embodiments, upon application of electrical current in the first direction to thermoelectric cooler 120, the temperature of external TEC face 124 is being elevated and the temperature of internal TEC face 122 is being lowered, thereby lowering the temperature of heat transferring medium 130. According to some embodiments, upon application of electrical current in the opposite direction to thermoelectric cooler 120, the temperature of external TEC face 124 is being lowered and the temperature of internal TEC face 122 is being elevated, thereby elevating the temperature of heat transferring medium 130. According to some embodiments, control unit 160 comprises an H-bridge configured to switch the direction of electrical current applied to thermoelectric cooler 120 between a first direction and an opposite direction, wherein upon application of electrical current in the first direction to thermoelectric cooler 120, the temperature of external TEC face 124 is being elevated and the temperature of internal TEC face 122 is being lowered, thereby lowering the temperature of heat transferring medium 130, and wherein upon application of electrical current in the opposite direction to thermoelectric cooler 120, the temperature of external TEC face 124 is being lowered and the temperature of internal TEC face 122 is being elevated, thereby elevating the temperature of heat transferring medium 130.

According to some embodiments, control unit 160 is configured to control the temperature of heat transferring medium 130 based on the temperature thereof. According to some embodiments, control unit 160 is configured to control the temperature of heat transferring medium 130 based on the temperature thereof, through monitoring the electrical current applied thereto. Control unit 160 may receive indication of the temperature of heat transferring medium 130 via signals from temperature sensor 162, as detailed below, according to some embodiments.

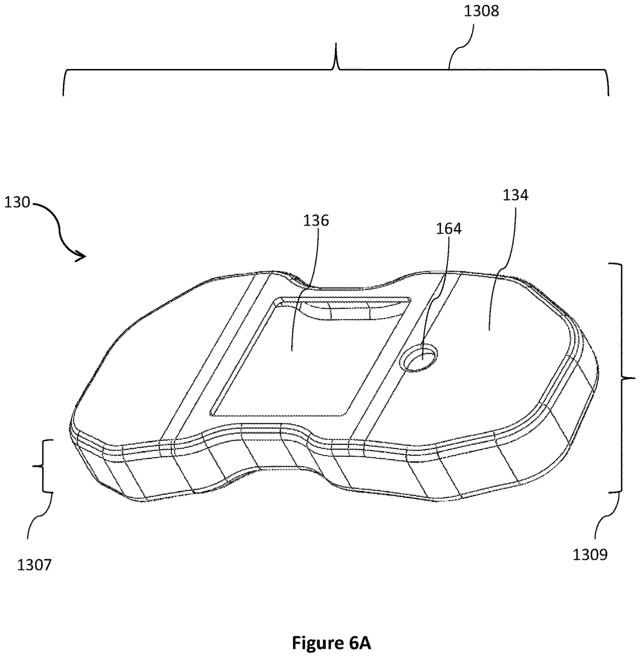

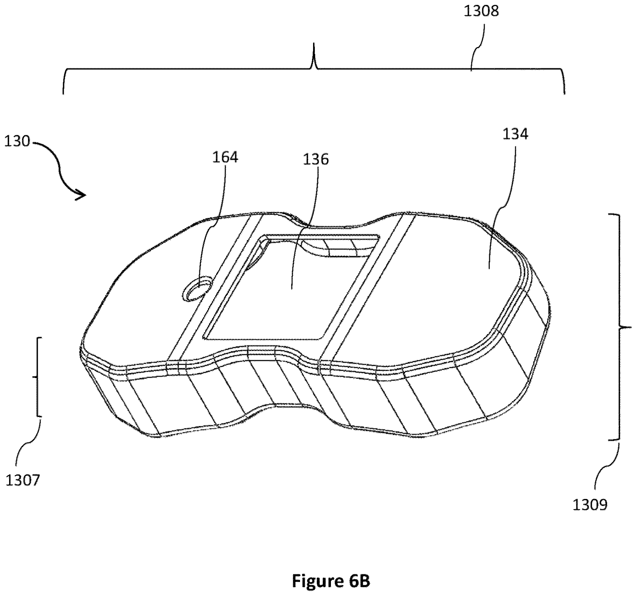

According to some embodiments, orthopedic brace 100 further comprises at least one temperature sensor 162. According to some embodiments, temperature sensor 162 is positioned in the vicinity of heat transferring medium 130. According to some embodiments, temperature sensor 162 is contacting heat transferring medium 130. According to some embodiments, heat transferring medium 130 comprises a temperature sensor slot 164. According to some embodiments, temperature sensor slot 164 is dimensioned to accommodate temperature sensor 162. According to some embodiments, temperature sensor 162 is accommodated within temperature sensor slot 164. Temperature sensor slot 164 may be seen in Figures 6A, 6B, 7A, 7B and 11. Via temperature sensor slot 164, temperature sensor 162 is capable of providing a more accurate indication of the inside temperature of heat transferring medium 130.

According to some embodiments, temperature sensor 162 is configured to measure the temperature of heat transferring medium 130. According to some embodiments, temperature sensor 162 is configured to send to control unit 160 temperature indicating signals indicative of the temperature of heat transferring medium 130. According to some embodiments, temperature sensor 162 is located within housing 102.

According to some embodiments, orthopedic brace 100 comprises a plurality of temperature sensors 162. It is to be understood than when plurality of temperature sensors 162 are used, each may function and/or be positioned as described above and as shown in the figures with one temperature sensor.

According to some embodiments, temperature sensor 162 may be an NTC temperature probe sensor. According to some embodiments, orthopedic brace 100 may comprise a plurality of temperature sensors 162, each configured to sense the temperature in one location heat transferring medium 130, housing 102 or in one location at the treated area.

According to some embodiments, orthopedic brace 100 comprises at least one sensor, other than a temperature sensor, configured to measure the subject's body status and reaction to the treatment (i.e. to application of orthopedic brace 100), such as, humidity (e.g. sweat), conductivity, and muscle movement among others.

According to some embodiments, control unit 160 comprises at least one predetermined operation program. By "comprises at least one predetermined operation program" it is intended to mean that control unit 160 has a computer readable instructions (program, software, firmware and the like) installed therein.

According to some embodiments, the operation program comprises instructions that when executed by control unit 160 bring the temperature of heat transferring medium 130 to a first predetermined temperature for a first predetermined period of time. According to some embodiments, to bring the temperature of heat transferring medium 130 to a first predetermined temperature entails controlling the current applied to thermoelectric cooler 120 by control unit 160 for an effective duration and intensity.

According to some embodiments, the operation program comprises instructions that when executed by control unit 160 bring the temperature of heat transferring medium 130 to a first predetermined temperature for a first predetermined period of time through controlling the current applied to thermoelectric cooler 120. According to some embodiments, the operation program comprises instruction that when executed by control unit 160 bring the temperature of heat transferring medium 130 to a first predetermined temperature for a first predetermined period of time through controlling the current applied to thermoelectric cooler 120 and based on the temperature indicating signals received from temperature sensor 162.

According to some embodiments, the first predetermined temperature is in the range of -5°C to 75°C. According to some embodiments, the first predetermined temperature is in the range of 0°C to 65°C. According to some embodiments, the first predetermined temperature is in the range of 5°C to 40°C.

According to some embodiments, control unit 160 is programmed for application of orthopedic brace 100 on the treated area for several cycles of treatment with non-treatment intervals. Optional treatment cycle times may range between 2 minutes and 120 minutes, followed by non-treatment intervals between 5 minutes and 24 hours. Other optional treatment cycle times are between 6 minutes and 30 minutes, with non-treatment intervals between 30 minutes and 2 hours. The treatment cycles can be cooling cycles, heating cycles and a combination thereof. Vibrating unit 168 may be activated during treatment cycles or non-treatment intervals, as detailed below.

One exemplary cooling pattern for the treatment of joint injury includes cooling to 10°C for 10 minutes, a 10 minutes non-cooling interval, cooling to 5°C for 20 minutes, a 10 minutes non-cooling interval, and cooling to 10°C for 20 minutes. Another exemplary cooling pattern for applying cold massage treatment includes cooling to 5°C for 20 minutes, a 10 minutes non-cooling interval with activation of therapeutic vibrating units 11, and repeating this cycle for 3-5 times. Another exemplary cooling/heating pattern for the treatment of osteoarthritis includes cooling to 5°C for 10 minutes, a 10 minutes non-cooling interval, heating to 40°C for 20 minutes, a 10 minutes non-heating interval, and cooling to 10°C for 20 minutes.

According to some embodiments, the program further comprises instructions that when executed by control unit 160 bring the temperature of heat transferring medium 130 to a second predetermined temperature for a second predetermined period of time. According to some embodiments, the program further comprises instructions that when executed by control unit 160 bring the temperature of heat transferring medium 130 to a second predetermined temperature for a second predetermined period of time through controlling the current applied to thermoelectric cooler 120. According to some embodiments, the program further comprises instructions that when executed by control unit 160 bring the temperature of heat transferring medium 130 to a second predetermined temperature for a second predetermined period of time through controlling the current applied to thermoelectric cooler 120 and based on the temperature indicating signals. According to some embodiments, the program further comprises bringing the temperature of heat transferring medium 130 to a second predetermined temperature for a second predetermined period of time through controlling the current applied to thermoelectric cooler 120 and based on the temperature indicating signals.

According to some embodiments, control unit 160 comprises a plurality of predetermined operation programs. According to some embodiments, each program comprises instructions that when executed by control unit 160 bring the temperature of heat transferring medium 130 to a predetermined temperature for a predetermined period of time. According to some embodiments, each program comprises instructions that when executed by control unit 160 bring the temperature of heat transferring medium 130 to a predetermined temperature for a predetermined period of time through controlling the current applied to thermoelectric cooler 120. According to some embodiments, each program comprises instructions that when executed by control unit 160 bring the temperature of heat transferring medium 130 to a predetermined temperature for a predetermined period of time through controlling the current applied to thermoelectric cooler 120 and based on the temperature indicating signals. According to some embodiments, control unit 160 comprises a plurality of predetermined operation programs, each program comprising bringing the temperature of heat transferring medium 130 to a predetermined temperature for a predetermined period of time through controlling the current applied to thermoelectric cooler 120 and based on the temperature indicating signals.

According to some embodiments, orthopedic brace 100 further comprises a control panel (not shown). According to some embodiments, the control panel is operatively coupled to control unit 160. The term "operatively coupled" refers to electronically or wirelessly coupled. The term "wirelessly coupled" refers to wireless connection as known in the art, i.e. involving communication between two electronic component via wireless signals.

According to some embodiments, the control panel is configured to send instruction signals to control unit 160. According to some embodiments, the instruction signals comprise: instructions to control the temperature of heat transferring medium 130, instructions to initiate at least one predetermined operation program, select one of the plurality of predetermined operation programs or a combination thereof. Each possibility represents a separate embodiment.

According to some embodiments, the control panel is connected to an outer surface of housing 102. According to some embodiments, the control panel is connected to internal housing face 104. It is to be understood that a control panel connected to orthopedic brace 100 may be electrically coupled to control unit 160, i.e. via electric wires.

According to some embodiments, control unit 160 is configured to send indication signals to the control panel. According to some embodiments, the control panel is configured to display indications indicated by said indication signals. According to some embodiments, the control panel comprises a display for displaying said indications.

According to some embodiments, the indication signals comprise: indication of the operation of the control unit 160, indication of the temperature of heat transferring medium 130, indication of a progression of a predetermined program or a combination thereof. Each possibility represents a separate embodiment.

According to some embodiments, orthopedic brace 100 comprises at least one LED lamp 166. According to some embodiments, at least one LED lamp 166 is externally visible through external housing face 106. According to some embodiments, at least one LED lamp 166 is electrically coupled with control unit 160. According to some embodiments, control unit 160 is configured to operate at least one LED lamp 166 to display at least one indication selected from the group consisting of: indication of the operation of the control unit 160, indication of the temperature of heat transferring medium 130, indication of a progression of a predetermined program or a combination thereof. Each possibility represents a separate embodiment. For example, at least one LED lamp 166 may turn on/off based on whether control unit 160 is operating. For another example, at least one LED lamp 166 may turn on/off, change its light intensity or color based on the temperature of heat transferring medium 130. For another example, at least one LED lamp 166 may turn on/off, change its light intensity or color based on the progression of the program, to indicate e.g. the time remaining. According to some embodiments, orthopedic brace 100 includes a plurality of LED lamps 166, each indicating at least one of said indications.

The term "plurality" refers to at least two of a specified element.

Although LED lamps 166 are shown in the figures, more complex visual elements, such as screens, are contemplated.

According to some embodiments, orthopedic brace 100 may further comprise one or more auditory elements configured to provide auditory information and/or feedback to the subject, e.g. information corresponding to the activity and/or status of the brace 100.

According to some embodiments, at least some of the information provided to the subject or to an operator of orthopedic brace 100 is provided through an external device. Such devices are known in the art and include, but not limited to, a smartphone wirelessly coupled to control unit 160, through a designate application, a desktop, a laptop, a tablet, a smart TV and the like.

According to some embodiments, control unit 160 is configured to receive wireless instruction signals from an external processing unit (not shown). According to some embodiments, the instruction signals comprise: instructions to control the temperature of heat transferring medium 130, instructions to initiate at least one predetermined operation select one of the plurality of predetermined operation programs or a combination thereof. Each possibility represents a separate embodiment. According to some embodiments, control unit 160 is configured to receive wireless instruction signals from an external processing unit, wherein the instruction signals comprise: instructions to control the temperature of heat transferring medium 130, instructions to initiate at least one predetermined operation select one of the plurality of predetermined operation programs or a combination thereof.

According to some embodiments, control unit 160 is configured to send wireless indication signals to the external processing unit. According to some embodiments, the processing unit is configured to display indications indicated by said indication signals through a display unit associated therewith. According to some embodiments, the indication signals comprise: indication of the operation of control unit 160, indication of the temperature of heat transferring medium 130, indication of a progression of a predetermined program and a combination thereof. Each possibility represents a separate embodiment. According to some embodiments, the external processing unit is configured to store the data received by the indication signals. According to some embodiments, the processing unit is configured to create a database from the stored data. According to some embodiments, the processing unit is configured to analyze the stored data.

According to some embodiments, control unit 160 comprises or is electrically coupled to a wireless receiver, configured to receive said wireless signals. According to some embodiments, control unit 160 comprises or is electrically coupled to a wireless transmitter, configured to transmit said wireless signals.

According to some embodiments, orthopedic brace 100 further comprises a vibrating unit 168. According to some embodiments, vibrating unit 168 is positioned on internal housing face 104.

According to some embodiments, vibrating unit 168 is positioned in the vicinity of subject's joint 116 when orthopedic brace 100 is worn by the subject. According to some embodiments, vibrating unit 168 is configured to provide vibration in order to massage (rub, press) the muscles, tendons or ligaments associated with subject's joint 116.

According to some embodiments, vibrating unit 168 is positioned in the vicinity of heat transferring medium 130. According to some embodiments, vibrating unit is contacting heat transferring medium 130. It is contemplated that when heat transferring medium 130 at least partially comprises a liquid in its first thermally conductive material, a contact between vibrating unit 168 and heat transferring medium 130 will facilitate convection within heat transferring medium 130 and will thus facilitate its reaching the desired temperature. Also, when heat transferring medium 130 is to be transformed from a bendable to a non-bendable state upon cooling, the convention assisted by vibrating unit 168 may facilitated the process, according to some embodiments.

According to some embodiments, vibrating unit 168 comprises a vibration motor, electrically coupled to control unit 160 and configured to create a vibration upon application of electric current thereby.

According to some embodiments, vibrating unit 168 is electrically coupled to control unit 160. According to some embodiments, vibrating unit 168 is operated by control unit 160. According to some embodiments, the program(s) installed on control unit 160 further comprises instructions that when executed by the control unit operate vibrating unit 168.

According to some embodiments, orthopedic brace 100 comprises a plurality of vibrating units 168. According to some embodiments, each one of plurality of vibrating units 168 individually may be characterized as detailed above.

Reference is now made to components of the cooling unit 170, which are shown in Figures 1A, 1B, 2A, 2B, 11, 12A, 12B and 13. Specifically, thermoelectric cooler 120 may cool or heat the heat transferring medium 130 and subject's joint 116 thereby, as elaborated above. When subject's joint 116 requires cooling, internal TEC face 122 is getting cold and external TEC face 124 is heated. In such cases, for proper operation and maintenance, external TEC face 124 is required to evacuate some of the excess heat created thereby. Cooling unit 170 is provided in order to address this challenge, according to some embodiments.

According to some embodiments, orthopedic brace 100 further comprises a cooling unit 170 configured to evacuate heat from external TEC face 124.

According to some embodiments, cooling unit 170 comprises a heat sink 172.

As used herein, the term "heat sink" will be understood to include devices and/or assemblies that serve to dissipate, carry away, or radiate heat generated by an active electronic device into the surrounding atmosphere.

According to some embodiments, heat sink 172 is coupled to thermoelectric cooler 120. According to some embodiments, heat sink 172 is thermally coupled to thermoelectric cooler 120. According to some embodiments, heat sink 172 is directly or indirectly connected to thermoelectric cooler 120. According to some embodiments, heat sink 172 is directly connected to thermoelectric cooler 120. According to some embodiments, heat sink 172 is facing external TEC face 124. According to some embodiments, heat sink 172 is directly connected to external TEC face 124.

According to some embodiments, cooling unit 170 comprises a plurality of heat sinks 172, each thermally coupled to thermoelectric cooler 120.

According to some embodiments, external TEC face 124 is connected to heat sink 172, through a second thermal paste 152 disposed there between. According to some embodiments, second thermal paste 152 has a thermal conductivity in the range of 0.5 to 100 $W*m^{-1}*K^{-1}$.

According to some embodiments, thermal paste 152 is a thermally conductive chemical compound, composed of mixture of thermally conductive materials in the form of paste, gel and liquid. According to some embodiments, thermal paste 152 is composed of at least one polymerizable liquid compound based on epoxides, silicones, urethanes, and/or acrylates, added with at least one metal oxide such as aluminum oxide and zinc oxide and/or metal nitride such as aluminum nitride and boron nitride. According to some embodiments, first thermal paste 150 may be a combination of one of the compositions described herein. According to some embodiments, thermal paste 150 comprises of at least one liquid metal/fusible metal. According to some embodiments, first thermal paste 150 and second thermal paste 152 have the same chemical composition. According to some embodiments, first thermal paste 150 and second thermal paste 152 have different chemical compositions.

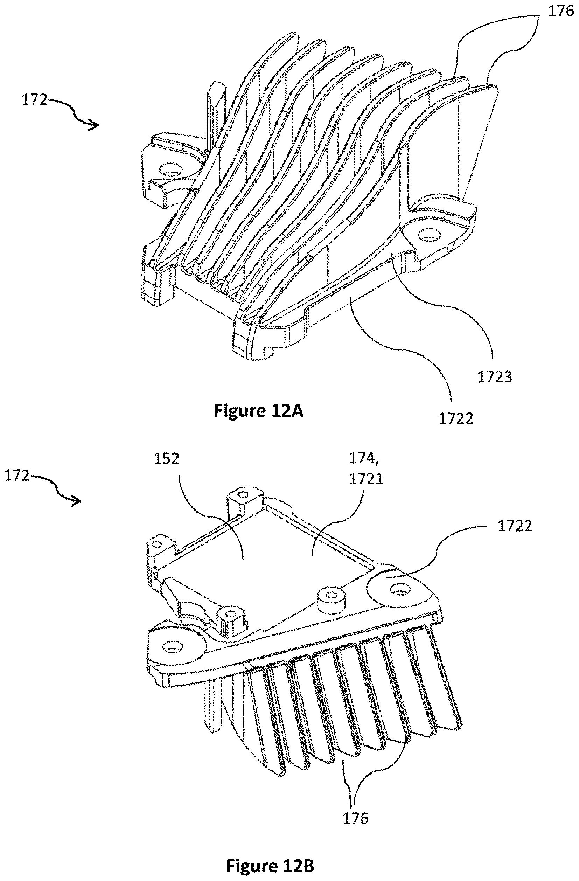

Reference is made to Figures 12A-B. According to some embodiments, heat sink 172 comprises a heat sink platform 1721 having a heat sink platform internal face 1722 and a heat sink platform external face 1723. According to some embodiments, heat sink platform internal face 1722 comprises a heat sink recess 174 dimensioned to accommodate external TEC face 124. According to some embodiments, external TEC face 124 is connected to heat sink recess 174. According to some embodiments, external TEC face 124 is connected to heat sink 172 through heat sink recess 174. According to some embodiments, external TEC face 124 is connected to heat sink recess 174 through a second thermal paste 152 disposed within heat sink recess 174.

According to some embodiments, heat sink 172 can be selected from passive or active heat sink types. In some embodiments, heat sink 172 is selected from the group consisting of extruded, stamped, bonded fin, folded fin, forged, swaged, single fin, skived and variations or combinations thereof

According to some embodiments, heat sink 172 further comprises a plurality of heat sink fins 176, each extending externally from heat sink platform external face 1723.

According to some embodiments, heat sink 172 is made of a heat conductive material. According to some embodiments, heat sink 172 is made of a heat conductive material having a thermal conductivity in the range of 7 to 24,000 $W*m^{-1}*K^{-1}$. According to some embodiments, the thermal conductivity of any one of these elements is preferably higher than 7.8 $W*m^{-1}*k^{-1}$.

According to some embodiments, heat sink 172 is made of a metal or metal alloy.

According to some embodiments, the metal or metal alloy is selected from the group consisting of Aluminum (Al), Beryllium (Be), Bismuth (Bi), Chromium (Cr), Cobalt (Co), Copper (Cu), Gallium (Ga), Gold (Au), Indium (In), Iron (Fe), Lead (Pb), Magnesium (Mg), Mercury (Hg), Nickel (Ni), Potassium (K), Rare Earths, Rhodium (Rh), Samarium (Sm), Scandium (Sc), Silver (Ag), Sodium (Na), Titanium (Ti), Tin (Sn), Zinc (Zn), and combinations thereof.

According to some embodiments, the Aluminum alloy is selected from the group consisting of aluminum, aluminum-lithium, aluminum-copper, aluminum-Scandium, aluminum-magnesium, aluminum-titanium, beryllium-aluminum, aluminum-gallium, aluminum-magnesium-manganese, aluminum-nickel-cobalt, aluminum-copper-iron-nickel, and aluminum-copper-nickel-magnesium alloy.

According to some embodiments, the Chromium alloy is a chromium-nickel or chromium-iron alloy.

According to some embodiments, the Cobalt alloy is a cobalt-chromium-molybdenum, cobalt-chromium-tungsten-carbon, cobalt-tungsten-molybdenum-carbon, cobalt-chromium-nickel-iron-molybdenum-tungsten, or cobalt-chromium-molybdenum alloy.

According to some embodiments, the copper alloy is a copper-zinc, copper-tin, copper-nickel, copper-aluminum, copper-gallium, copper-tungsten, copper-silver, copper-gold, copper-lead, cooper-beryllium, copper-silver-gold, copper-tin-zinc, cooper-beryllium-iron, copper-nickel-iron, copper-nickel-manganese copper-aluminum-zinc, copper-indium-gallium, or copper-aluminum-zinc-tin alloy.

According to some embodiments, the gold alloy is a gold-copper, gold-silver, gold-rhodium, gold-iron, gold-silver-copper, gold-iron-copper, or gold-nickel-palladium alloy.

According to some embodiments, the iron alloy is an iron-carbon (carbon steel), iron-carbon-molybdenum, manganese, chromium or nickel (low alloy steel), iron-carbon-chromium (stainless steel), iron-carbon-chromium-nickel, iron-carbon-tungsten, iron-carbon-cobalt, iron-carbon-tungsten-cobalt, iron-carbon-manganese, or iron-gallium alloy.

According to some embodiments, the nickel alloy is a nickel-chromium, nickel-iron, nickel-carbon, nickel-silicon, nickel-molybdenum, nickel-aluminum, nickel-copper, nickel-titanium, nickel-cobalt, nickel-gallium, nickel-aluminum-cobalt, nickel-chromium-iron, nickel-copper-zinc, nickel-chromium-iron, nickel-iron-molybdenum, nickel-titanium-aluminum, nickel-copper-zinc-manganese, nickel-copper-iron-manganese, nickel-chromium-molybdenum-tungsten, nickel-chromium-silicon-magnesium, or nickel-chromium-cobalt-titanium alloy.

According to some embodiments, the silver alloy is a silver-copper, silver-gold, silver-platinum, silver-copper-gold, or silver-copper-germanium alloy.

According to some embodiments, the titanium alloy is a titanium-aluminum, titanium-gold, titanium-vanadium, titanium-aluminum-vanadium, or titanium-vanadium-chromium alloy.

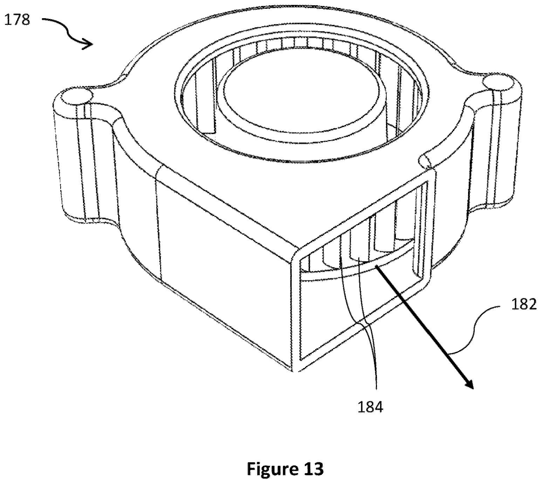

According to some embodiments, cooling unit 170 further comprises a fan 178 configured and positioned to create air flow 182 in the direction of heat sink 172, thereby evacuating heat therefrom. According to some embodiments, fan 178 comprises a plurality of fan blades 184, shaped to create air flow 182 in the direction of heat sink 172. Specifically fan 178 is shown separately in Figure 13 and in relation to heat sink 172 in Figure 11. The direction of air flow 182 is indicated by an arrow as can be appreciated in these Figures.

The term "fan" as used herein in interchangeable with any one of the terms "ventilator" or "blower".

According to some embodiments, air flow 182 in the direction of heat sink 172 is flowing through plurality of heat sink fins 176 and exits orthopedic brace 100 through a first ventilation outlet 196. According to some embodiments, first ventilation outlet 196 comprises at least one opening in housing 102. According to some embodiments, first ventilation outlet 196 is positioned in the vicinity of heat sink 172.

According to some embodiments, housing 102 further comprises second ventilation outlet 180 in the vicinity of fan 178.

According to some embodiments, control unit 160 is electrically coupled to fan 178. According to some embodiments, control unit 160 is configured to control fan 178. According to some embodiments, the program installed on control unit 160 further comprises instructions, that when executed by control unit 160 operate or cease operation of fan 178. According to some embodiments, the program installed on control unit 160 further comprises instructions, that when executed by control unit 160 operate or cease operation of fan 178 based on whether electric current applied to thermoelectric cooler 120 is causing external TEC face 124 to heat or to cool. According to some embodiments, the program installed on control unit 160 further comprises instructions to operate fan 178 by control unit 160 when external TEC face 124 is being heated by the current applied to thermoelectric cooler 120.

Reference is now made to Figures 2A, 2B, 7A, 7B and 8.

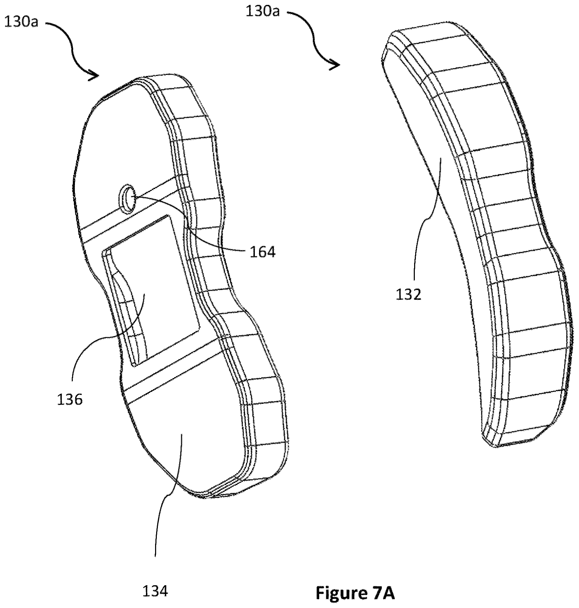

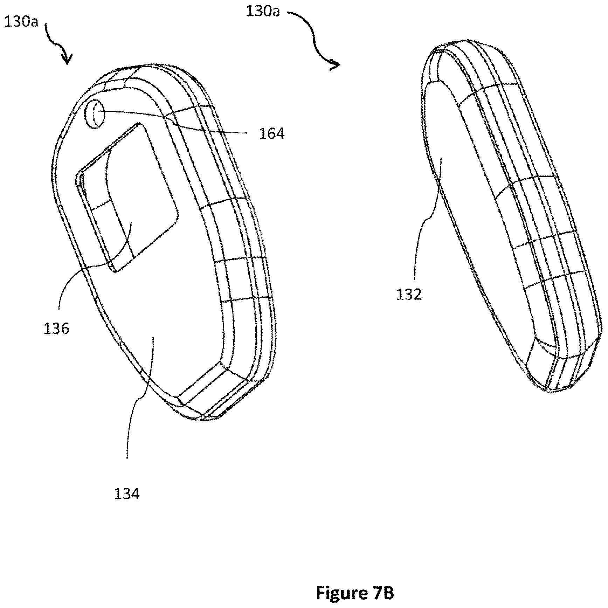

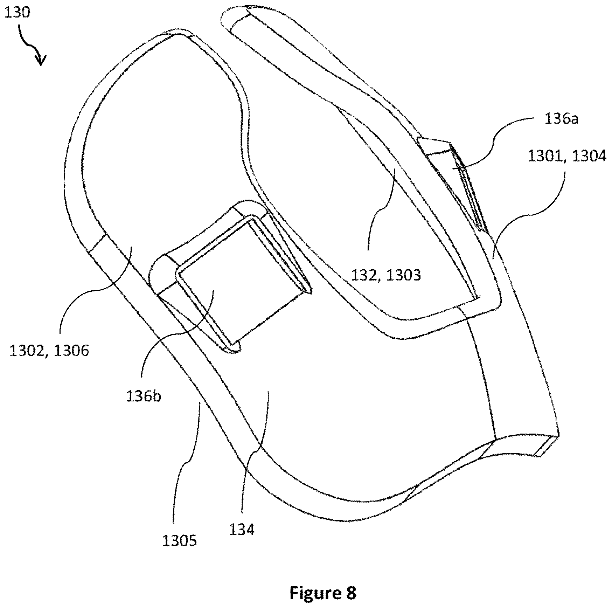

Specifically, Figures 2A-B show views in perspective of orthopedic braces when worn on a subject's leg, according to some embodiments. These Figures are shown orthopedic brace 100 including two separate housings 102 (housing 102a and housing 102b), each comprising a part of orthopedic brace 100, and connected by strap 192. In Figures 2A and 2B each of the housings may include any element of orthopedic brace 100 as disclosed herein as shown with respect to housing 102a, which is easily visible, according to some embodiments. Specifically, each one of housing 102a and housing 102b includes thermoelectric cooler 120, heat transferring medium 130, optionally heat transferring connector 140 and cooling unit 170, including their various components. Control unit 160, as can be appreciated be the skilled in the art, may still be a single unit operating component associated with either housing 102a or housing 102b. However, the present configuration of orthopedic brace 100 is not limited to a single control unit 160. More specifically, Figures 2A-B show a configuration of orthopedic brace 100, in which two separate heat transferring media 130 are included. This is also shown in Figures 7A and 7B. However, a single heat transferring medium 130 may extend between housing 102a and housing 102b as shown in Figure 8 and as detailed herein.

According to some embodiments, orthopedic brace 100 comprises a first housing 102a having a first internal housing face 104a and a first external housing face 106a. According to some embodiments, first internal housing face 104a is structured to be worn by the subject, in the vicinity of subject's joint 116. According to some embodiments, orthopedic brace 100 further comprises a second housing 102b having a second internal housing face 104b and a second external housing face 106b, wherein internal housing face 104b is structured to be worn by the subject, in the vicinity of subject's joint 116. Each of said elements is elaborated above, when referring to housing 102, internal housing face 104 and external housing face 106.

According to some embodiments, orthopedic brace 100 further comprises a first substantially flat thermoelectric cooler (TEC) 120a. According to some embodiments, thermoelectric cooler 120a is disposed at least partially within first housing 102a. According to some embodiments, thermoelectric cooler 120a has a first internal TEC face 122a and a first external TEC face 124a. According to some embodiments, first external TEC face 124a is facing external housing face 106a. According to some embodiments, orthopedic brace 100 further comprises a second substantially flat thermoelectric cooler (TEC) 120b. According to some embodiments, thermoelectric cooler 120b is disposed at least partially within second housing 102b. According to some embodiments, thermoelectric cooler 120b is having a second internal TEC face 122b and a second external TEC face 124b. According to some embodiments, second external TEC face 124b is facing external housing face 106b. Each of said elements is elaborated above, when referring to thermoelectric cooler 120, internal TEC face 122 and external TEC face 124.

According to some embodiments, orthopedic brace 100 comprises at least one heat transferring medium 130 thermally coupled to at least one of first thermoelectric cooler 120a and second thermoelectric cooler 120b. According to some embodiments, heat transferring medium 130 comprises a first thermally conductive material. According to some embodiments, at least one heat transferring medium 130 has a heat transferring medium external face 134 and heat transferring medium internal face 132. According to some embodiments, heat transferring medium external face 134 is facing and thermally coupled to at least one of first internal TEC face 122a and second internal TEC face 122b. Each of said elements is elaborated above, when referring to heat transferring medium 130, heat transferring medium internal face 132 and heat transferring medium external face 134.

According to some embodiments, upon application of electrical current to each one of first thermoelectric cooler 120a and second thermoelectric cooler 120b, the temperature of the corresponding external TEC face (124a or 124b) is being either elevated or lowered and the temperature of the corresponding internal TEC face (122a or 122b) is being either lowered or elevated in the opposite direction, thereby lowering or elevating the temperature of heat transferring medium 130 thermally coupled thereto.

With respect to the usage of the term "corresponding" above, it is to be understood that external TEC face 124a, internal TEC face 122a and thermoelectric cooler 120a are corresponding one to the other, and that external TEC face 124b, internal TEC face 122b and thermoelectric cooler 120b are separately corresponding one to the other.

According to some embodiments, orthopedic brace 100 comprises a first heat transferring medium 130a and a second heat transferring medium 130b. According to some embodiments, first heat transferring medium 130a is coupled to first thermoelectric cooler 120a. According to some embodiments, first heat transferring medium 130a comprises a thermally conductive material. According to some embodiments, first heat transferring medium 130a has a first heat transferring medium external face 134a, facing and thermally coupled to first internal TEC face 122a, and a first heat transferring medium internal face 132a. According to some embodiments, second heat transferring medium 130b is coupled to second thermoelectric cooler 120b. According to some embodiments, second heat transferring medium 130b comprises a thermally conductive material. According to some embodiments, second heat transferring medium 130b has a second heat transferring medium external face 134b, facing and thermally coupled to second internal TEC face 122b, and a second heat transferring medium internal face 132b. According to some embodiments, upon application of electrical current to first thermoelectric cooler 120a, the temperature of first external TEC face 124a is being either elevated or lowered and the temperature of first internal TEC face 122a is being either lowered or elevated in the opposite direction, thereby lowering or elevating the temperature of first heat transferring medium 130a. According to some embodiments, upon application of electrical current to second thermoelectric cooler 120b, the temperature of second external TEC face 124b is being either elevated or lowered and the temperature of second internal TEC face 124b is being either lowered or elevated in the opposite direction, thereby lowering or elevating the temperature of the second heat transferring medium 130a. The configuration of the current paragraph is shown in Figures 2A and 2B. other features of this configuration are described and can be understood, e.g. from the description of various configurations of orthopedic brace 100 herein, such as the configuration shown in Figures 1A and 1B.

According to some embodiments, orthopedic brace 100 comprises a single heat transferring medium 130 extending between first housing 102a and second housing 102b. According to some embodiments, single heat transferring medium 130 has a heat transferring medium first portion 1301 coupled to first thermoelectric cooler 120a and a heat transferring medium second portion 1302 coupled to second thermoelectric cooler 120b. According to some embodiments, single heat transferring medium 130 comprises a first thermally conductive material. According to some embodiments, heat transferring medium first portion 1301 has a heat transferring medium first portion external face 1304, facing and thermally coupled to first internal TEC face 122a. According to some embodiments, heat transferring medium first portion 1301 has a heat transferring medium first portion internal face 1303. According to some embodiments, heat transferring medium second portion 1302 has a heat transferring medium second portion external face 1306, facing and thermally coupled to second internal TEC face 122b. According to some embodiments, heat transferring medium second portion 1302 has a heat transferring medium second portion internal face 1305. According to some embodiments, upon application of electrical current to each one of first thermoelectric cooler 120a and second thermoelectric cooler 120b, the temperature of the corresponding external TEC face (124a or 124b) is being either elevated or lowered and the temperature of the corresponding internal TEC face (122a or 122b) is being either lowered or elevated in the opposite direction, thereby lowering or elevating the temperature of the heat transferring medium first portion 1301 or heat transferring medium second portion 1302, coupled thereto. Single heat transferring medium 130 of the configuration of the current paragraph is shown in Figure 8.

According to some embodiments, orthopedic brace 100 comprises a control unit 160 disposed within one of first housing 102a and second housing 102b. According to some embodiments, control unit 160 is electrically coupled to each one of first thermoelectric cooler 120a and second thermoelectric cooler 120b and configured to monitor the electrical current applied thereto. According to some embodiments, orthopedic brace 100 comprises a control unit 160 disposed within one of first housing 102a and second housing 102b, wherein control unit 160 is electrically coupled to each one of first thermoelectric cooler 120a and second thermoelectric cooler 120b and configured to monitor the electrical current applied thereto. According to some embodiments, a single control unit 160 is electrically coupled to each one of first thermoelectric cooler 120a and second thermoelectric cooler 120b, wherein at least one of first thermoelectric cooler 120a and second thermoelectric cooler 120b is electrically coupled to control unit 160 via an electric wire extending through cable stress relief 194 (shown in figures 1A and 11).

As seen in Figures 2A-B orthopedic brace 100 may further include at least one connective belt configured to fasten attachment of housings 102a and 102b to the subject body part 118.

According to some embodiments, first housing 102a comprises a first strap holder 190a, extending externally from external housing face 106a According to some embodiments, second housing 102b comprises a second strap holder 190b, extending externally from external housing face 106b.

According to some embodiments, orthopedic brace 100 further comprises a strap 192 configured to connect between first housing 102a and second housing 102b through first strap holder 190a and second strap holder 190b, and further configured to adjust orthopedic brace 100 to be worn by the subject. According to some embodiments, each one of first housing 102a and second housing 102b comprises a cable stress relief 194 for connecting electric wires between housing 102a and 102b of the orthopedic brace 100. As detailed above, such cables may be used for electronic communication between control unit 160 and electronic elements disposed in a housing (102a or 102b) in which control unit 160 is not disposed.

According to some embodiments, there is provided a method for treating a disorder, disease or an injury of a joint of a subject, wherein the joint is selected from the group consisting of a knee joint, an elbow joint, a wrist joint, an ankle joint, a shoulder joint, a hip joint, a spine joint, a finger j oint, and a toe joint, the method comprising applying orthopedic brace 100 of the present invention onto subj ect's joint 116.

According to some embodiments the orthopedic brace 100 can be combined with other therapeutic technologies including, but not limited to, pulsed ultrasound treatment, electromagnetic field treatment, transcutaneous electrical nerve stimulation, and low-level laser therapy.

According to some embodiments, there is provided a method of treating joint disorders, diseases or injuries using orthopedic brace 100. The orthopedic brace 100 can be used to change or alter the functioning of the musculoskeletal system. It can be additionally, or alternatively, used for rehabilitation from illness, trauma, and even in congenital conditions.

The orthopedic brace 100 can be used to support and control the joints of a subject in need thereof. The orthopedic brace 100 can be used on the ankle, knee, hip, back, neck, elbow, fingers, and wrist. It can be used to control the position of limbs and initiate specific movement or motion in the body. Also, the brace 100 may compensate for weak muscles and correct structural deformities. The orthopedic braces 100 can be used for diagnostic application.

Thus, according to some embodiments, there is provided a method for diagnosing a disease, the disease is selected from arthritis, osteoarthritis, rheumatoid arthritis, osteoporosis, cerebral palsy, spina bifida, spinal cord injury, scoliosis, using brace 100.

According to some embodiments, there is provided a method for treating and/or handling after stroke effects using brace 100.

The orthopedic brace 100 can also be used after knee joint surgery in order to keep the joints immobile and control motion until the joint recovers. The brace 100 can also be used to provide extra support to prevent injuries to joints. The orthopedic brace 100 can be used for preventive treatment, namely, to prevent injury to a joint with a high load. Athletes, such as football and rugby players, can use the active brace 100 to prevent injuries.

The orthopedic brace 100 can also be used by arthritis and trauma patients to improve their functionality. Advantageously, brace 100 is easy to use and hence is most suitable for treating the rising population of geriatric individuals who are susceptible to musculoskeletal disorders, such as, osteoarthritis, osteoporosis and related fractures. In addition, brace 100 provides a suitable solution to needs related to sport activities such as injuries and trauma. Also, the use of orthopedic brace 100 such as knee, elbow and ankle braces by athletes as a preventive measure can reduce the load on the joint and prevent injuries.

The heat transferring medium 130 can also be applied for climate control by cooling/heating outer body surfaces such as one's head, neck, shoulders, thorax (chest), abdomen (stomach), pelvis, arms and legs, in the form of wearable clothing such as a helmet, collar, suit, shirt, trousers, shoes, or gloves.

The following examples are to be considered merely as illustrative and non-limiting in nature. It will be apparent to one skilled in the art to which the present invention pertains that many modifications, permutations, and variations may be made without departing from the scope of the invention.

EXAMPLE 1

TEC Thermal study of an active orthopedic brace

A thermal study was conducted on the thermal system of an active orthopedic brace to estimate the heat transfer and its cooling efficiency.

The thermal study was conducted on components of the thermal system shown in Fig. 7, which included TEC module 04908 (Hebei I.T. Shanghai), with heat level set at the maximum theoretical output of 30 W, where the temperature at the cold side can be reduced down to -5°C. As detailed below, the thermal study shows thermal efficiency at ambient and external temperatures of 25°C, 30°C and 35°C. For each temperature, two scenarios were studied, one with the fan "on" and one with the fan "off". The results are summarized in Tables 1, 2 and 3.

Table 1: Thermal efficiency at ambient temperature of 25°C

| Component | Component Temperature (°C) | |
|---|---|---|
| Fan | off | on |
| TEC (hot side) | 38 | 38 |
| TEC-Tube connector | 38 | 32 |
| Heat tube | 36 | 30 |
| Heat sink | 30-31 | 24-25 |

At ambient temperature of 25°C, at maximal TEC efficiency, the maximal heat temperature of the TEC-tube connector in passive state is 38°C, where upon fan activation the temperature was reduced to 32°C at the connector, and to 25°C at the heat sink.

At external temperature of 30°C, at maximal TEC efficiency, the maximal heat temperature of the TEC-tube connector in passive state was 43°C, and upon fan activation the temperature was reduced to 37°C at the connector, and to 30°C at the heat sink.

Table 2: Thermal efficiency at external temperature of 30°C

| Component | Component Temperature (°C) | |
|---|---|---|
| Fan | off | on |
| TEC (hot side) | 43 | 43 |
| TEC-Tube connector | 43 | 37 |
| Heat tube | 41 | 34 |
| Heat sink | 36-37 | 30 |

Table 3: Thermal efficiency at external temperature of 35°C

| Component | Component Temperature (°C) | |
|---|---|---|
| Fan | off | on |
| TEC (hot side) | 48 | 48 |
| TEC-Tube connector | 48 | 42 |
| Heat tube | 45-46 | 40 |
| Heat sink | 40-41 | 34-35 |

At external temperature of 35°C, at maximal TEC efficiency, the maximal heat temperature of the TEC-tube connector in passive state was 48°C, where upon fan activation the temperature was reduced to 42°C at the connector, and to 34-35°C at the heat sink.

The aforementioned thermal qualities of the thermal system indicate that the thermal system is suitable for working with medical equipment, and specifically for use on the human body. Nevertheless, the system can be improved by optimization of the heat conduction system.

It should be understood that the above description is merely exemplary and that there are various embodiments of the present invention that may be devised, mutatis mutandis, and that the features described in the above-described embodiments, and those not described herein, may be used separately or in any suitable combination; and the invention can be devised in accordance with embodiments not necessarily described above.

It is understood that aspect and embodiments described herein include "consisting" and/or "consisting essentially of" aspects and embodiments. As used herein, the singular form "a", "an", and "the" includes plural references unless indicated otherwise.

While this invention has been disclosed with reference to specific embodiments, it is apparent that other embodiments and variations of this invention may be devised by others skilled in the art

**Claims**

1. An orthopedic brace (100) comprising:

   at least one housing (102) having an internal face (104) and an external face (106), wherein said internal face (104) is structured to be worn by a subject, in a vicinity of a joint;
   at least one substantially flat thermoelectric cooler (TEC) (120) disposed at least partially within the at least one housing, and having an internal TEC face (122) and an external TEC face (124), wherein the external TEC face is facing the external face (106) of the at least one housing (102);
   at least one heat transferring medium (130) coupled to the thermoelectric cooler (120) and comprising a first thermally conductive material, wherein the heat transferring medium has an external face (134), facing and thermally coupled to the internal TEC face (122), and an internal face (132); and
   a cooling unit (170) comprising:

      a heat sink (172) coupled to the thermoelectric cooler, and
      a fan (178) configured and positioned to create air flow in the direction of the heat sink, thereby evacuating heat therefrom
      wherein upon application of electrical current to the thermoelectric cooler (120), the temperature of the external TEC face (124) is being either elevated or lowered and the temperature of the internal TEC face (122) is being either lowered or elevated in the opposite direction, thereby lowering or elevating the temperature of the heat transferring medium; **characterized in that**,
      lowering the temperature of the heat transferring medium comprises setting the temperature of heat transferring medium at a first temperature, wherein the heat transferring medium is in a non-bendable state when in the first temperature.

2. The orthopedic brace of claim 1, wherein the heat transferring medium is in a bendable state when in room temperature, and wherein lowering the temperature of the heat transferring medium to the first temperature causes the heat transferring medium to be in a non-bendable state.

3. The orthopedic brace of claim 2, wherein when the orthopedic brace is worn by the subject and when in the heat transferring medium is in a non-bendable state, the joint is substantially fixed in a predetermined conformation; and wherein when the orthopedic brace is worn by the subject and when in the heat transferring medium is in a bendable state, the joint is substantially movable.

4. The orthopedic brace of claim 1, further comprising a heat transferring connector (140) made of a second thermally conductive material, the heat transferring connector having an external face (144) coupled to the at least one thermoelectric cooler and an internal face (142) contacting the heat transferring medium, wherein the heat transferring medium comprises a niche (136), the niche located at the external face of the heat transferring medium and penetrating into the heat transferring medium, and
wherein the niche is configured to accommodate at least a portion of the heat transferring connector.

5. The orthopedic brace of claim 4, wherein a part of the heat transferring connector is located within the niche of the heat transferring medium.

6. The orthopedic brace of claim 5, wherein the part of the heat transferring connector is positioned to be in direct contact with the internal TEC face.

7. The orthopedic brace of any one of claims 1-6, wherein upon application of electrical current to the thermoelectric cooler, the temperature of the internal TEC face is being either lowered or elevated, thereby lowering or elevating the temperature of the heat transferring connector respectively, and wherein upon the temperature of the heat transferring connector being either lowered or elevated, heat is conducted between the heat transferring connector and the heat transferring medium to lower or elevate the temperature of the heat transferring medium respectively.

8. The orthopedic brace of any one of claims 1-7, wherein the heat sink comprises a platform having an internal face and an external face, wherein the internal face comprises a recess dimensioned to accommodate the external TEC face, wherein the internal heat sink face is connected to the recess, through a second thermal paste disposed therein, wherein the heat sink further comprises a plurality of fins, each extending externally from the external heat sink face.

9. The orthopedic brace of claim 1, further comprising a vibrating unit (168) positioned on the internal face of the at least one housing;
wherein the vibrating unit is contacting the heat transferring medium.

10. The orthopedic brace of claim 10, wherein the vibrating unit comprises a vibration motor, electrically coupled to the control unit and configured to create a vibration upon application of electric current thereby.

11. The orthopedic brace of claim 9 or 10, wherein the first thermally conductive material comprises a liquid.

12. The orthopedic brace of claim 9 or 10, wherein the heat transferring medium is in a bendable state when in room temperature, and wherein the lowering of the temperature of the heat transferring medium to the first temperature causes the heat transferring medium to be in a non-bendable state.

13. The orthopedic brace of claim 1, wherein the at least one housing comprises

a first housing (102a), wherein the internal face of the first housing is structured to be worn by the subject, in the vicinity of the joint;
a second housing (102b), wherein the internal face of the second housing is structured to be worn by the subject, in the vicinity of the same joint;
wherein the at least one substantially flat thermoelectric cooler comprises:

a first substantially flat thermoelectric cooler (120a) disposed at least partially within the first housing, wherein the external TEC face is facing the external face of the first housing (102a);
a second substantially flat thermoelectric cooler (120b) disposed at least partially within the second housing, wherein the external TEC face of the second thermoelectric cooler (120b) is facing the external face of the second housing; and
wherein the at least one heat transferring medium comprises:

a single heat transferring medium extending between the first housing and the second housing and having a first portion coupled to the first thermoelectric cooler and a second portion coupled to the second thermoelectric cooler, wherein the single heat transferring medium comprises a first thermally conductive material, wherein the first portion has a first portion external face, facing and thermally coupled to the first internal TEC face and a first portion internal face, wherein the second portion has a second portion external face, facing and thermally coupled to the second internal TEC face and a second portion internal face;
wherein upon application of electrical current to each one of the first thermoelectric cooler and second thermoelectric cooler, the temperature of the corresponding external TEC face is being either elevated or lowered and the temperature of the corresponding internal TEC face is being either lowered or elevated in the opposite direction, thereby lowering or elevating the temperature of the first or second portion, coupled thereto.

14. The orthopedic brace of any one of claims 1-13, indicated to treat the joint of a subject.


**Patentansprüche**

1. Orthese (100), welche umfasst:

mindestens ein Gehäuse (102) mit einer Innenfläche (104) und einer Außenfläche (106), worin die Innenfläche (104) so strukturiert ist, dass sie von einer Person in der Nähe eines Gelenks getragen werden kann;
mindestens einen im Wesentlichen flachen thermoelektrischen Kühler (TEC) (120), der zumindest teilweise innerhalb des mindestens einen Gehäuses angeordnet ist und eine innere TEC-Fläche (122) und eine äußere TEC-Fläche (124) aufweist, worin die äußere TEC-Fläche der Außenfläche (106) des mindestens einen Gehäuses (102) zugewandt ist;
mindestens ein Wärmeübertragungsmedium (130), das mit dem thermoelektrischen Kühler (120) gekoppelt ist und ein erstes wärmeleitendes Material umfasst, worin das Wärmeübertragungsmedium eine Außenfläche (134), die der inneren TEC-Fläche (122) zugewandt und mit dieser thermisch gekoppelt ist, und eine Innenfläche (132) aufweist; und
eine Kühleinheit (170), die umfasst:

einen Kühlkörper (172), der mit dem thermoelektrischen Kühler gekoppelt ist, und
ein Gebläse (178), das ausgestaltet und angeordnet ist, einen Luftstrom in Richtung des Kühlkörpers zu erzeugen, wodurch Wärme von diesem abgeführt wird;

worin beim Anlegen von elektrischem Strom an den thermoelektrischen Kühler (120) die Temperatur der äußeren TEC-Fläche (124) entweder erhöht oder gesenkt wird und die Temperatur der inneren TEC-Fläche (122) in der entgegengesetzten Richtung entweder gesenkt oder erhöht wird, wodurch die Temperatur des Wärmeübertragungsmediums gesenkt oder erhöht wird;
**dadurch gekennzeichnet, dass**,

das Absenken der Temperatur des Wärmeübertragungsmediums das Einstellen der Temperatur des Wärmeübertragungsmediums auf eine erste Temperatur umfasst, worin das Wärmeübertragungsmedium in einem nicht biegbaren Zustand ist, wenn es die erste Temperatur hat.

2. Orthese nach Anspruch 1,
worin sich das Wärmeübertragungsmedium bei Raumtemperatur in einem biegbaren Zustand befindet, und worin das Absenken der Temperatur des Wärmeübertragungsmediums auf die erste Temperatur bewirkt, dass sich das Wärmeübertragungsmedium in einem nicht biegbaren Zustand befindet.

3. Orthese nach Anspruch 2, wobei, wenn die Orthese von der Person getragen wird und wenn sie sich in dem Wärmeübertragungsmedium in einem nicht biegbaren Zustand befindet, das Gelenk im Wesentlichen in einer bestimmten Form fixiert ist; und wobei, wenn die Orthese von der Person getragen wird und wenn sie sich in dem Wärmeübertragungsmedium in einem biegbaren Zustand befindet, das Gelenk im Wesentlichen beweglich ist.

4. Orthese nach Anspruch 1, welche ferner ein wärmeübertragendes Verbindungsstück (140) aus einem zweiten wärmeleitenden Material umfasst, worin das wärmeübertragende Verbindungsstück eine Außenfläche (144), die mit dem mindestens einen thermoelektrischen Kühler verbunden ist, und eine Innenfläche (142) aufweist, die das Wärmeübertragungsmedium berührt,

worin das Wärmeübertragungsmedium eine Nische (136) umfasst, worin die Nische an der Außenfläche des Wärmeübertragungsmediums angeordnet ist und in das Wärmeübertragungsmedium eindringt, und
worin die Nische ausgestaltet ist, mindestens einen Teil des wärmeübertragenden Verbindungsstücks aufzunehmen.

5. Orthese nach Anspruch 4, worin ein Teil des wärmeübertragenden Verbindungsstücks innerhalb der Nische des Wärmeübertragungsmediums angeordnet ist.

6. Orthese nach Anspruch 5, worin der Teil des wärmeübertragenden Verbindungsstücks angeordnet ist, in direktem Kontakt mit der inneren TEC-Fläche zu stehen.

7. Orthese nach einem der Ansprüche 1-6,
worin beim Anlegen von elektrischem Strom an den thermoelektrischen Kühler die Temperatur der inneren TEC-Fläche entweder gesenkt oder erhöht wird, wodurch die Temperatur des wärmeübertragenden Verbindungsstücks gesenkt bzw. erhöht wird, und worin beim Senken oder Erhöhen der Temperatur des wärmeübertragenden Verbindungsstücks Wärme zwischen dem wärmeübertragenden Verbindungsstück und dem Wärmeübertragungsmedium geleitet wird, um die Temperatur des Wärmeübertragungsmediums zu senken bzw. zu erhöhen.

8. Orthese nach einem der Ansprüche 1-7, worin der Kühlkörper eine Plattform mit einer Innenfläche und einer Außenfläche umfasst, worin die Innenfläche eine Aussparung umfasst, die bemessen ist, die externe TEC-Fläche aufzunehmen, worin die innere Kühlkörperfläche mit der Aussparung durch eine zweite darin angeordnete Wärmeleitpaste verbunden ist, worin der Kühlkörper ferner mehrere Rippen umfasst, die sich jeweils von der äußeren Kühlkörperfläche nach außen erstrecken.

9. Orthese nach Anspruch 1, welche ferner mit einer Vibrationseinheit (168) umfasst, die an der Innenfläche des mindestens einen Gehäuses angeordnet ist; worin die Vibrationseinheit mit dem Wärmeübertragungsmedium in Kontakt ist.

10. Orthese nach Anspruch 9, worin die vibrierende Einheit einen Vibrationsmotor umfasst, der mit der Steuereinheit elektrisch gekoppelt und ausgestaltet ist, bei Anlegen von elektrischem Strom eine Vibration zu erzeugen.

11. Orthese nach Anspruch 9 oder 10, worin das erste wärmeleitende Material eine Flüssigkeit umfasst.

12. Orthese nach Anspruch 9 oder 10, worin sich das Wärmeübertragungsmedium bei Raumtemperatur in einem biegbaren Zustand befindet und worin das Absenken der Temperatur des Wärmeübertragungsmediums auf die erste Temperatur bewirkt, dass sich das Wärmeübertragungsmedium in einem nicht-biegbaren Zustand befindet.

13. Orthese nach Anspruch 1 , worin das mindestens eine Gehäuse umfasst:

ein erstes Gehäuse (102a), worin die Innenfläche des ersten Gehäuses so strukturiert ist, dass dieses von der Person in der Nähe des Gelenks getragen werden kann; ein zweites Gehäuse (102b), worin die Innenfläche des zweiten Gehäuses so strukturiert ist, dass dieses von der Person in der Nähe desselben Gelenks getragen werden kann; worin der mindestens eine im Wesentlichen flache thermoelektrische Kühler umfasst:

einen ersten im Wesentlichen flachen thermoelektrischen Kühler (120a), der zumindest teilweise innerhalb des ersten Gehäuses angeordnet ist, worin die äußere TEC-Fläche der Außenfläche des ersten Gehäuses (102a) zugewandt ist; einen zweiten im Wesentlichen flachen thermoelektrischen Kühler (120b), der zumindest teilweise innerhalb des zweiten Gehäuses angeordnet ist, worin die äußere TEC-Fläche des zweiten thermoelektrischen Kühlers (120b) der Außenfläche des zweiten Gehäuses zugewandt ist; und worin das mindestens eine Wärmeübertragungsmedium umfasst:

ein einzelnes Wärmeübertragungsmedium, das sich zwischen dem ersten Gehäuse und dem zweiten Gehäuse erstreckt und einen ersten Abschnitt, der mit dem ersten thermoelektrischen Kühler gekoppelt ist, und einen zweiten Abschnitt, der mit dem zweiten thermoelektrischen Kühler gekoppelt ist, aufweist, worin das einzelne Wärmeübertragungsmedium ein erstes wärmeleitendes Material umfasst, worin der erste Abschnitt eine Außenfläche des ersten Abschnitts, die der ersten inneren TEC-Fläche zugewandt und mit dieser thermisch gekoppelt ist, und eine Innenfläche des ersten Abschnitts aufweist, worin der zweite Abschnitt eine Außenfläche des zweiten Abschnitts, die der zweiten inneren TEC-Fläche zugewandt und mit dieser thermisch gekoppelt ist, und eine Innenfläche des zweiten Abschnitts aufweist; wobei beim Anlegen von elektrischem Strom an den ersten thermoelektrischen Kühler und den zweiten thermoelektrischen Kühler die Temperatur der entsprechenden externen TEC-Fläche entweder erhöht oder gesenkt wird und die Temperatur der entsprechenden internen TEC-Fläche in der entgegengesetzten Richtung entweder gesenkt oder erhöht wird, wodurch die Temperatur des ersten oder zweiten Teils, der damit verbunden ist, gesenkt oder erhöht wird.

14. Orthese nach einem der Ansprüche 1 bis 13, welche zur Behandlung des Gelenks eines Patienten geeignet ist.

## Revendications

1. Une orthèse orthopédique (100) comprenant:

au moins un boîtier (102) ayant une face interne (104) et une face externe (106), dans lequel ladite face interne (104) est structurée pour être portée par un sujet, à proximité d'une articulation;

au moins un refroidisseur thermoélectrique (TEC) sensiblement plat (120) disposé au moins partiellement à l'intérieur du boîtier au moins, et ayant une face TEC interne (122) et une face TEC externe (124), la face TEC externe faisant face à la face externe (106) du boîtier au moins (102);

au moins un fluide caloporteur (130) couplé au refroidisseur thermoélectrique (120) et comprenant un premier matériau thermoconducteur, dans lequel le fluide caloporteur présente une face externe (134), orientée vers la face TEC interne (122) et couplée thermiquement à celle-ci, et une face interne (132); et

une unité de refroidissement (170) comprenant un dissipateur thermique (172) couplé au refroidisseur thermoélectrique, et

un ventilateur (178) configuré et positionné pour créer un flux d'air dans la direction du dissipateur thermique, évacuant ainsi la chaleur de celui-ci dans lequel, lors de l'application d'un courant électrique au refroidisseur thermoélectrique (120), la température de la face externe de la TEC (124) est soit élevée soit abaissée et la température de la face interne de la TEC (122) est soit abaissée soit élevée dans la direction opposée, abaissant ou élevant ainsi la température du fluide caloporteur;

**caractérisé par le fait que**

l'abaissement de la température du fluide caloporteur consiste à régler la température du fluide caloporteur à une première température, le fluide caloporteur étant dans un état non pliable lorsqu'il est à la première température.

2. Orthèse orthopédique de la revendication 1, dans laquelle le fluide caloporteur est dans un état pliable lorsqu'il est à température ambiante, et dans laquelle l'abaissement de la température du fluide caloporteur à la première température fait que le fluide caloporteur est dans un état non pliable.

3. Orthèse orthopédique de la revendication 2, dans laquelle, lorsque l'orthèse orthopédique est portée par le sujet et que le fluide caloporteur est dans un état non pliable, l'articulation est sensiblement fixe dans une conformation prédéterminée; et dans laquelle, lorsque l'orthèse orthopédique est portée par le sujet et que le fluide caloporteur est dans un état pliable, l'articulation est sensiblement mobile.

4. Orthèse orthopédique de la revendication 1, comprenant en outre un connecteur de transfert de chaleur (140) constitué d'un second matériau thermoconducteur, le connecteur de transfert de chaleur ayant une face externe (144) couplée à l'au moins un refroidisseur thermoélectrique et une face interne (142) en contact avec le fluide caloporteur,

dans lequel le fluide caloporteur comprend une niche (136), la niche étant située sur la face externe du fluide caloporteur et pénétrant dans le fluide caloporteur, et

dans laquelle la niche est configurée pour accueillir au moins une partie du connecteur de transfert de chaleur.

5. Orthèse orthopédique de la revendication 4, dans laquelle une partie du connecteur de transfert de chaleur est située dans la niche du fluide caloporteur.

6. Orthèse orthopédique de la revendication 5, dans laquelle la partie du connecteur de transfert de chaleur est positionnée de manière à être en contact direct avec la face interne du TEC.

7. Orthèse orthopédique de l'une des revendications 1 à 6,

dans laquelle, lors de l'application d'un courant électrique au refroidisseur thermoélectrique, la température de la face interne du TEC est abaissée ou élevée, abaissant ou élevant ainsi la température du connecteur de transfert de chaleur, et dans laquelle, lorsque la température du connecteur de transfert de chaleur est abaissée ou élevée, la chaleur est conduite entre le connecteur de transfert de chaleur et le fluide caloporteur pour abaisser ou élever la température du fluide caloporteur, respectivement.

8. Orthèse orthopédique de l'une des revendications 1 à 7,

dans lequel le dissipateur thermique comprend une plate-forme ayant une face interne et une face externe, dans lequel la face interne comprend un renfoncement dimensionné pour accueillir la face externe du TEC, dans lequel la face interne du dissipateur thermique est reliée au renfoncement, par l'intermédiaire d'une seconde pâte thermique disposée dans celui-ci, dans lequel le dissipateur thermique comprend en outre une pluralité d'ailettes, chacune s'étendant à l'extérieur de la face externe du dissipateur thermique.

9. Orthèse orthopédique de la revendication 1, comprenant en outre une unité vibrante (168) positionnée sur la face interne de l'au moins un boîtier; dans laquelle l'unité vibrante est en contact avec le fluide caloporteur.

10. Orthèse orthopédique de la revendication 10, dans laquelle l'unité vibrante comprend un moteur vibrant, couplé électriquement à l'unité de commande et configuré pour créer une vibration lors de l'application d'un courant électrique.

11. Orthèse orthopédique de la revendication 9 ou 10, dans laquelle le premier matériau thermoconducteur comprend un liquide.

12. Orthèse orthopédique de la revendication 9 ou 10, dans laquelle le fluide caloporteur est dans un état pliable lorsqu'il est à température ambiante, et dans laquelle l'abaissement de la température du fluide caloporteur à la première température fait que le fluide caloporteur est dans un état non pliable.

13. Orthèse orthopédique de la revendication 1, dans laquelle l'au moins un boîtier comprend:

un premier boîtier (102a), dans lequel la face interne du premier boîtier est structurée pour être portée par le sujet, à proximité de l'articulation;
un second boîtier (102b), dans lequel la face interne du second boîtier est structurée pour être portée par le sujet, à proximité de la même articulation;
dans lequel l'au moins un refroidisseur thermoélectrique sensiblement plat comprend:

un premier refroidisseur thermoélectrique sensiblement plat (120a) disposé au moins partiellement à l'intérieur du premier boîtier, dans lequel la face externe TEC fait face à la face externe du premier boîtier (102a);
un second refroidisseur thermoélectrique sensiblement plat (120b) disposé au moins partiellement à l'intérieur du second boîtier, dans lequel la face TEC externe du second refroidisseur thermoélectrique (120b) fait face à la face externe du second boîtier; et
dans lequel l'au moins un fluide caloporteur comprend:

un fluide caloporteur unique s'étendant entre le premier boîtier et le second boîtier et ayant une première partie couplée au premier refroidisseur thermoélectrique et une seconde partie couplée au second refroidisseur thermoélectrique, dans lequel le fluide caloporteur unique comprend un premier matériau thermoconducteur, dans lequel la première partie a une première face externe, faisant face et couplée thermiquement à la première face TEC interne et à une première face interne, dans lequel la seconde partie a une seconde face externe, faisant face et couplée thermiquement à la seconde face TEC interne et à une seconde face interne;
dans lequel, lors de l'application d'un courant électrique à chacun du premier et du second refroidisseur thermoélectrique, la température de la face externe correspondante de la TEC est soit élevée, soit abaissée, et la température de la face interne correspondante de la TEC est soit abaissée, soit élevée dans la direction opposée, abaissant ou élevant ainsi la température de la première ou de la seconde partie, couplée à celle-ci.

14. Orthèse orthopédique de l'une des revendications 1 à 13, indiquée pour traiter l'articulation d'un sujet.

**Figure 1A**

**Figure 1B**

**Figure 2A**

**Figure 2B**

126b

126a

120

124

122

140, 148

140, 146

134

136

130

**Figure 3A**

**Figure 3B**

**Figure 4**

**Figure 5A**

**Figure 5B**

1308

130

136

164

134

1307

1309

**Figure 6A**

**Figure 6B**

**Figure 7A**

130a

130a

164

136

132

134

**Figure 7B**

**Figure 8**

120

124

126b

126a

122

**Figure 9**

144

140

**Figure 10A**

142

140

144

**Figure 10B**

142

**Figure 11**

**Figure 12A**

**Figure 12B**

178

182

184

**Figure 13**

## REFERENCES CITED IN THE DESCRIPTION

**Patent documents cited in the description**

- WO 02000159 A
- WO 03090868 A
- US 20180056069 A
- US 10226640 B
- US 5314455 A
- US 5466250 A
- US 7243509 B
- US 8043242 B
- US 9808395 B
- WO 2005007060 A
- US 20140046232 A
- WO 2015079237 A
- US 20190015289 A
- WO 2018102182 A
- WO 20180138537 A